# EUROPEAN PATENT SPECIFICATION

(11) **EP 2 539 344 B1**
(45) Date of publication and mention of the grant of the patent: **15.10.2014**
(21) Application number: 11706530.0
(22) Date of filing: 25.02.2011
(51) Int. Cl.: C07D 495/04, A61K 31/519, A61P 3/00, A61P 3/06, A61P 3/10, A61P 13/12, A61P 25/28, A61P 29/00, A61P 35/00

(54) **HALOGEN OR CYANO SUBSTITUTED THIENO[2,3-D]PYRIMIDINES HAVING MNK1/ MNK2 INHIBITING ACTIVITY FOR PHARMACEUTICAL COMPOSITIONS**
HALOGEN ODER CYANO SUBSTITUIERTE THIENO[2,3-D]PYRIMIDINE MIT MNK1/MNK2-HEMMENDER WIRKUNG FÜR PHARMAZEUTISCHE ZUSAMMENSETZUNGEN
THIÉNO[2,3-D]PYRIMIDINES SUBSTITUÉES PAR UN HALOGÈNE OU UN CYANO AYANT UNE ACTIVITÉ INHIBITRICE MNK1/MNK2 UTILISÉES DANS DES COMPOSITIONS PHARMACEUTIQUES

(30) Priority: 26.02.2010 EP 10154928
(43) Date of publication of application: 02.01.2013
(73) Proprietor: Boehringer Ingelheim International GmbH, 55216 Ingelheim am Rhein (DE)
(72) Inventor: HECKEL, Armin, 55216 Ingelheim Am Rhein (DE); HIMMELSBACH, Frank, 55216 Ingelheim Am Rhein (DE); LEHMANN-LINTZ, Thorsten, 55216 Ingelheim Am Rhein (DE); REDEMANN, Norbert, 55216 Ingelheim Am Rhein (DE); SAUER, Achim, 55216 Ingelheim Am Rhein (DE); THOMAS, Leo, 55216 Ingelheim Am Rhein (DE); BLACK, Phillip, Saffron Walden Essex CB10 1XL (GB); BLACKABY, Wesley, Saffron Walden Essex CB10 1XL (GB); DANILEWICZ, John, Ash Canterbury Kent CT3 2AF (GB); LINNEY, Ian, Saffron Walden Essex CB10 1XL (GB); AUSTEN, Matthias, 37079 Goettingen (DE); SCHNEIDER, Martin, 37079 Goettingen (DE); SCHREITER, Kay, 37079 Goettingen (DE)
(74) Representative: Simon, Elke Anna Maria
(86) International application number: PCT/EP2011/052811
(87) International publication number: WO 2011/104338

(56) References cited:
- WO-A1-2006/136402
- WO-A1-2010/023181
- WO-A2-2007/059905

## Description

The present invention relates to thienopyrimidine compounds and to novel pharmaceutical compositions comprising thienopyrimidine compounds.

Moreover, the present invention relates to the use of the thienopyrimidine compounds of the invention for the production of pharmaceutical compositions for the prophylaxis and/or treatment of diseases which can be influenced by the inhibition of the kinase activity of Mnk1 (Mnk1a or MnK1b) and/or Mnk2 (Mnk2a or Mnk2b) or further variants thereof. Particularly, the present invention relates to the use of the thienopyrimidine compounds of the invention for the production of pharmaceutical compositions for the prophylaxis and/or therapy of metabolic diseases, such as diabetes, hyperlipidemia and obesity, hematopoietic disorders, neurodegenerative diseases, kidney damage, inflammatory disorders, and cancer and their consecutive complications and disorders associated therewith.

Metabolic diseases are diseases caused by an abnormal metabolic process and may either be congenital due to an inherited enzyme abnormality or acquired due to a disease of an endocrine organ or failure of a metabolically important organ such as the liver or the pancreas.

The present invention is more particularly directed to the treatment and/or prophylaxis of in particular metabolic diseases of the lipid and carbohydrate metabolism and the consecutive complications and disorders associated therewith.

Lipid disorders cover a group of conditions which cause abnormalities in the level and metabolism of plasma lipids and lipoproteins. Thus, hyperlipidemias are of particular clinical relevance since they constitute an important risk factor for the development of atherosclerosis and subsequent vascular diseases such as coronary heart disease.

Diabetes mellitus is defined as a chronic hyperglycemia associated with resulting damages to organs and dysfunctions of metabolic processes. Depending on its etiology, one differentiates between several forms of diabetes, which are either due to an absolute (lacking or decreased insulin secretion) or to a relative lack of insulin. Diabetes mellitus Type I (IDDM, insulin-dependent diabetes mellitus) generally occurs in adolescents under 20 years of age. It is assumed to be of auto-immune etiology, leading to an insulitis with the subsequent destruction of the beta cells of the islets of Langerhans which are responsible for the insulin synthesis. In addition, in latent autoimmune diabetes in adults (LADA; Diabetes Care. 8: 1460-1467, 2001) beta cells are being destroyed due to autoimmune attack. The amount of insulin produced by the remaining pancreatic islet cells is too low, resulting in elevated blood glucose levels (hyperglycemia). Diabetes mellitus Type II generally occurs at an older age. It is above all associated with a resistance to insulin in the liver and the skeletal muscles, but also with a defect of the islets of Langerhans. High blood glucose levels (and also high blood lipid levels) in turn lead to an impairment of beta cell function and to an increase in beta cell apoptosis.

Diabetes is a very disabling disease, because today's common anti-diabetic drugs do not control blood sugar levels well enough to completely prevent the occurrence of high and low blood sugar levels. Out of range blood sugar levels are toxic and cause long-term complications for example retinopathy, renopathy, neuropathy and peripheral vascular disease. There is also a host of related conditions, such as obesity, hypertension, heart disease and hyperlipidemia, for which persons with diabetes are substantially at risk.

Obesity is associated with an increased risk of follow-up diseases such as cardiovascular diseases, hypertension, diabetes, hyperlipidemia and an increased mortality. Diabetes (insulin resistance) and obesity are part of the "metabolic syndrome" which is defined as the linkage between several diseases (also referred to as syndrome X, insulin-resistance syndrome, or deadly quartet). These often occur in the same patients and are major risk factors for development of diabetes type II and cardiovascular disease. It has been suggested that the control of lipid levels and glucose levels is required to treat diabetes type II, heart disease, and other occurrences of metabolic syndrome (see e.g., Diabetes 48: 1836-1841, 1999; JAMA 288: 2209-2716, 2002).

In one embodiment of the present invention the compounds and compositions of the present invention are useful for the treatment and/or prophylaxis of metabolic diseases of the carbohydrate metabolism and their consecutive complications and disorders such as impaired glucose tolerance, diabetes (preferably diabetes type II), diabetic complications such as diabetic gangrene, diabetic arthropathy, diabetic osteopenia, diabetic glomerosclerosis, diabetic nephropathy, diabetic dermopathy, diabetic neuropathy, diabetic cataract and diabetic retinopathy, diabetic maculopathy, diabetic feet syndrome, diabetic coma with or without ketoacidosis, diabetic hyperosmolar coma, hypoglycemic coma, hyperglycemic coma, diabetic acidosis, diabetic ketoacidosis, intracapillary glomerulonephrosis, Kimmelstiel-Wilson syndrome, diabetic amyotrophy, diabetic autonomic neuropathy, diabetic mononeuropathy, diabetic polyneuropathy, diabetic angiopathies, diabetic peripheral angiopathy, diabetic ulcer, diabetic arthropathy, or obesity in diabetes.

In a further embodiment the compounds and compositions of the present invention are useful for the treatment and/or prophylaxis of metabolic diseases of the lipid metabolism (i.e. lipid disorders) and their consecutive complications and disorders such as hypercholesterolemia, familial hypercholesterolemia, Fredrickson's hyperlipoproteinemia, hyperbetalipoproteinemia, hyperlipidemia, low-density-lipoprotein-type [LDL] hyperlipoproteinemia, pure hyperglyceridemia, endogenous hyperglyceridemia, isolated hypercholesterolemia, isolated hypertroglyceridemia, cardiovascular diseases such as hypertension, ischemia, varicose veins, retinal vein occlusion, atherosclerosis, angina pectoris, myocardial infarction, stenocardia, pulmonary hypertension, congestive heart failure, glomerulopaty, tubulointestitial disorders, renal failure, angiostenosis, or cerebrovascular disorders, such as cerebral apoplexy.

In a further embodiment of the present invention the compounds and compositions of the present invention are useful for the treatment and/or prophylaxis of hematopoetic disorders and their consecutive complications and disorders such as acute myeloid leukemia (AML), Morbus Hodgkin, Non-Hodgkin's lymphoma; hematopoetic disease, acute non-lymphocytic leukemia (ANLL), myeloproliferative disease acute promyelocytic leukemia (APL), acute myelomonocytic leukemia (AMMoL), multiple myeloma, polycythemia vera, lymphoma, acute lymphocytic leukemia (ALL), chronic lymphocytic leukemia (CCL), Wilm's tumor, or Ewing's Sarcoma.

In a further embodiment of the present invention the compounds and compositions of the present invention are useful for the treatment and/or prophylaxis of cancer and consecutive complications and disorders such as cancer of the upper gastrointestinal tract, pancreatic carcinoma, breast cancer, colon cancer, ovarian carcinoma, cervix carcinoma, endometrial cancer, brain tumor, testicular cancer, laryngeal carcinoma, osteocarcinoma, prostatic cancer, retinoblastoma, liver carcinoma, lung cancer, neuroblastoma, renal carcinoma, thyroid carcinoma, esophageal cancer, soft tissue sarcoma, skin cancer, osteosarcoma, rhabdomyosarcoma, bladder cancer, metastatic cancer, cachexia, or pain.

Certain anti-cancer drugs such as cisplatin are linked to serious side effects such as nephrotoxicity or ototoxicity, which can be dose limiting. Activation of Mnks has been linked to these side effects. In a further embodiment of the present invention, the compounds and compositions of the present invention are useful for the treatment and/or prophylaxis of ear or kidney damage, in particular for the prevention or treatment of ear and kidney drug induced damage

Furthermore, the present invention relates to the use of thienopyrimidine compounds for the production of pharmaceutical compositions for the prophylaxis and/or therapy of cytokine related diseases.

Such diseases are i.a. inflammatory diseases, autoimmune diseases, destructive bone disorders, proliferative disorders, infectious diseases, neurodegenerative diseases, allergies, or other conditions associated with proinflammatory cytokines. Allergic and inflammatory diseases such as acute or chronic inflammation, chronic inflammatory arthritis, rheumatoid arthritis, psoriasis, COPD, inflammatory bowel disease, asthma and septic shock and their consecutive complications and disorders associated therewith.

Inflammatory diseases like rheumatoid arthritis, inflammatory lung diseases like COPD, inflammatory bowel disease and psoriasis afflict one in three people in the course of their lives. Not only do those diseases impose immense health care costs, but also they are often crippling and debilitating.

Although inflammation is the unifying pathogenic process of these inflammatory diseases below, the current treatment approach is complex and is generally specific for any one disease. Many of the current therapies available today only treat the symptoms of the disease and not the underlying cause of inflammation.

The compositions of the present invention are useful for the treatment and/or prophylaxis of inflammatory diseases and consecutive complications and disorders. such as chronic or acute inflammation, inflammation of the joints such as chronic inflammatory arthritis, rheumatoid arthritis, psoriatic arthritis, osteoarthritis, juvenile rheumatoid arthritis, Reiter's syndrome, rheumatoid traumatic arthritis, rubella arthritis, acute synovitis and gouty arthritis; inflammatory skin diseases such as sunburn, psoriasis, erythrodermic psoriasis, pustular psoriasis, eczema, dermatitis, acute or chronic graft formation, atopic dermatitis, contact dermatitis, urticaria and scleroderma; inflammation of the gastrointestinal tract such as inflammatory bowel disease, Crohn's disease and related conditions, ulcerative colitis, colitis, and diverticulitis; nephritis, urethritis, salpingitis, oophoritis, endomyometritis, spondylitis, systemic lupus erythematosus and related disorders, multiple sclerosis, asthma, meningitis, myelitis, encephalomyelitis, encephalitis, phlebitis, thrombophlebitis, respiratory diseases such as asthma, bronchitis, chronic obstructive pulmonary disease (COPD), inflammatory lung disease and adult respiratory distress syndrome, and allergic rhinitis; endocarditis, osteomyelitis, rheumatic fever, rheumatic pericarditis, rheumatic endocarditis, rheumatic myocarditis, rheumatic mitral valve disease, rheumatic aortic valve disease, prostatitis, prostatocystitis, spondoarthropathies ankylosing spondylitis, synovitis, tenosynovotis, myositis, pharyngitis, polymyalgia rheumatica, shoulder tendonitis or bursitis, gout, pseudo gout, vasculitides, inflammatory diseases of the thyroid selected from granulomatous thyroiditis, lymphocytic thyroiditis, invasive fibrous thyroiditis, acute thyroiditis; Hashimoto's thyroiditis, Kawasaki's disease, Raynaud's phenomenon, Sjogren's syndrome, neuroinflammatory disease, sepsis, conjunctivitis, keratitis, iridocyclitis, optic neuritis, otitis, lymphoadenitis, nasopaharingitis, sinusitis, pharyngitis, tonsillitis, laryngitis, epiglottitis, bronchitis, pneumonitis, stomatitis, gingivitis. oesophagitis, gastritis, peritonitis, hepatitis, cholelithiasis, cholecystitis, glomerulonephritis, goodpasture's disease, crescentic glomerulonephritis, pancreatitis, endomyometritis, myometritis, metritis, cervicitis, endocervicitis, exocervicitis, parametritis, tuberculosis, vaginitis, vulvitis, silicosis, sarcoidosis, pneumoconiosis, pyresis, inflammatory polyarthropathies, psoriatric arthropathies, intestinal fibrosis, bronchiectasis and enteropathic arthropathies.

Moreover, cytokines are also believed to be implicated in the production and development of various cardiovascular and cerebrovascular disorders such as congestive heart disease, myocardial infarction, the formation of atherosclerotic plaques, hypertension, platelet aggregation, angina, stroke, Alzheimer's disease, reperfusion injury, vascular injury including restenosis and peripheral vascular disease, and, for example, various disorders of bone metabolism such as osteoporosis (including senile and postmenopausal osteoporosis), Paget's disease, bone metastases, hypercalcaemia, hyperparathyroidism, osteosclerosis, osteoporosis and periodontitis, and the abnormal changes in bone metabolism which may accompany rheumatoid arthritis and osteoarthritis.

Excessive cytokine production has also been implicated in mediating certain complications of bacterial, fungal and/or viral infections such as endotoxic shock, septic shock and toxic shock syndrome and in mediating certain complications of CNS surgery or injury such as neurotrauma and ischaemic stroke.

Excessive cytokine production has, moreover, been implicated in mediating or exacerbating the development of diseases involving cartilage or muscle resorption, pulmonary fibrosis, cirrhosis, renal fibrosis, the cachexia found in certain chronic diseases such as malignant disease and acquired immune deficiency syndrome (AIDS), tumour invasiveness and tumour metastasis and multiple sclerosis. The treatment and/or prophylaxis of these diseases are also contemplated by the present invention

Additionally, the inventive compositions may be used to treat inflammation associated with autoimmune diseases including, but not limited to, systemic lupus erythematosis, Addison's disease, autoimmune polyglandular disease (also known as autoimmune polyglandular syndrome), glomerulonephritis, rheumatoid arthritis scleroderma, chronic thyroiditis, Graves' disease, autoimmune gastritis, diabetes, autoimmune hemolytic anemia, glomerulonephritis, rheumatoid arthritis autoimmune neutropenia, thrombocytopenia, atopic dermatitis, chronic active hepatitis, myasthenia gravis, multiple sclerosis, inflammatory bowel disease, ulcerative colitis, Crohn's disease, psoriasis, and graft vs. host disease.

In a further embodiment the compositions of the present invention may be used for the treatment and prevention of infectious diseases such as sepsis, septic shock, Shigellosis, and Helicobacter pylori and viral diseases including herpes simplex type 1 (HSV-1), herpes simplex type 2 (HSV-2), cytomegalovirus, Epstein-Barr, human immunodeficiency virus (HIV), acute hepatitis infection (including hepatitis A, hepatits B, and hepatitis C), HIV infection and CMV retinitis, AIDS or malignancy, malaria, mycobacterial infection and meningitis. These also include viral infections, by influenza virus, varicella-zoster virus (VZV), Epstein-Barr virus, human herpesvirus-6 (HHV-6), human herpesvirus-7 (HHV-7), human herpesvirus-8 (HHV-8), Poxvirus, Vacciniavirus, Monkeypoxvirus, pseudorabies and rhinotracheitis.

The compositions of the present invention may also be used topically in the treatment or prophylaxis of topical disease states mediated by or exacerbated by excessive cytokine production, such as inflamed joints, eczema, psoriasis and other inflammatory skin conditions such as sunburn; inflammatory eye conditions including conjunctivitis; pyresis, pain and other conditions associated with inflammation.

Periodontal disease has also been implemented in cytokine production, both topically and systemically. Hence, use of compositions of the present invention to control the inflammation associated with cytokine production in such peroral diseases such as gingivitis and periodontitis is another aspect of the present invention.

Finally, the compositions of the present invention may also be used to treat or prevent neurodegenerative disease selected from Alzheimer's disease, Parkinson's disease, amyotrophic lateral sclerosis, Huntington's disease, frontotemporal lobar dementia, spinocerebellar ataxia, dementia with Lewy bodies, cerebral ischemia or neurodegenerative disease caused by traumatic injury, glutamate neurotoxicity or hypoxia.

In a preferred embodiment the compositions of the present invention may be used to treat or prevent a disease selected from chronic or acute inflammation, chronic inflammatory arthritis, rheumatoid arthritis, psoriasis, COPD, inflammatory bowel disease, septic shock, Crohn's disease, ulcerative colitis, multiple sclerosis and asthma.

Protein kinases are important enzymes involved in the regulation of many cellular functions. The LK6-serine/threonine-kinase gene of Drosophila melanogaster was described as a short-lived kinase which can associate with microtubules (J. Cell Sci. 1997, 110(2): 209-219). Genetic analysis in the development of the compound eye of Drosophila suggested a role in the modulation of the RAS signal pathway (Genetics 2000 156(3): 1219-1230). The closest human homologues of Drosophila LK6-kinase are the MAP-kinase interacting kinase 2 (Mnk2, e.g. the variants Mnk2a and Mnk2b) and MAP-kinase interacting kinase 1 (Mnk1) and variants thereof. These kinases are mostly localized in the cytoplasm. Mnks are phosphorylated by the p42 MAP kinases Erk1 and Erk2 and the p38-MAP kinases. This phosphorylation is triggered in a response to growth factors, phorbol esters and oncogenes such as Ras and Mos, and by stress signaling molecules and cytokines. The phosphorylation of Mnk proteins stimulates their kinase activity towards eukaryotic initiation factor 4E (elF4E) (EMBO J. 16: 1909-1920, 1997; Mol Cell Biol 19, 1871-1880, 1990; Mol Cell Biol 21, 743-754, 2001). Simultaneous disruption of both, the Mnk1 and Mnk2 gene in mice diminishes basal and stimulated elF4E phosphorylation (Mol Cell Biol 24, 6539-6549, 2004). Phosphorylation of elF4E results in a regulation of the protein translation (Mol Cell Biol 22: 5500-5511, 2001).

There are different hypotheses describing the mode of the stimulation of the protein translation by Mnk proteins. Most publications describe a positive stimulatory effect on the cap-dependent protein translation upon activation of MAP kinase-interacting kinases. Thus, the activation of Mnk proteins can lead to an indirect stimulation or regulation of the protein translation, e.g. by the effect on the cytosolic phospholipase 2 alpha (BBA 1488:124-138, 2000).

WO 03/037362 discloses a link between human Mnk genes, particularly the variants of the human Mnk2 genes, and diseases which are associated with the regulation of body weight or thermogenesis. It is postulated that human Mnk genes, particularly the Mnk2 variants are involved in diseases such as e.g. metabolic diseases including obesity, eating disorders, cachexia, diabetes mellitus, hypertension, coronary heart disease, hypercholesterolemia, dyslipidemia, osteoarthritis, biliary stones, cancer of the genitals and sleep apnea, and in diseases connected with the ROS defense, such as e.g. diabetes mellitus and cancer. WO 03/03762 moreover discloses the use of nucleic acid sequences of the MAP kinase-interacting kinase (Mnk) gene family and amino acid sequences encoding these and the use of these sequences or of effectors of Mnk nucleic acids or polypeptides, particularly Mnk inhibitors and activators in the diagnosis, prophylaxis or therapy of diseases associated with the regulation of body weight or thermogenesis.

WO 02/103361 describes the use of kinases 2a and 2b (Mnk2a and Mnk2b) interacting with the human MAP kinase in assays for the identification of pharmacologically active ingredients, particularly useful for the treatment of diabetes mellitus type 2. Moreover, WO 02/103361 discloses also the prophylaxis and/or therapy of diseases associated with insulin resistance, by modulation of the expression or the activity of Mnk2a or Mnk2b. Apart from peptides, peptidomimetics, amino acids, amino acid analogues, polynucleotides, polynucleotide analogues, nucleotides and nucleotide analogues, 4-hydroxybenzoic acid methyl ester are described as a substance which binds the human Mnk2 protein.

First evidence for a role of Mnks in inflammation was provided by studies demonstrating activation of Mnk1 by proinflammatory stimuli. The cytokines TNFα and IL-1β trigger the activation of Mnk1 in vitro (Fukunaga and Hunter, EMBO J 16(8): 1921-1933, 1997) and induce the phosphorylation of the Mnk-specific substrate elF4E in vivo (Ueda et al., Mol Cell Biol 24(15): 6539-6549, 2004). In addition, administration of lipopolysaccharide (LPS), a potent stimulant of the inflammatory response, induces activation of Mnk1 and Mnk2 in mice, concomitant with a phosphorylation of their substrate elF4E (Ueda et al., Mol Cell Biol 24(15): 6539-6549, 2004).

Furthermore, Mnk1 has been shown to be involved in regulating the production of proinflammatory cytokines. Mnk1 enhances expression of the chemokine RANTES (Nikolcheva et al., J Clin Invest 110, 119-126, 2002). RANTES is a potent chemotractant of monocytes, eosinophils, basophiles and, natural killer cells. It activates and induces proliferation of T lymphocytes, mediates degranulation of basophils and induces the respiratory burst in eosinophils (Conti and DiGioacchino, Allergy Asthma Proc 22(3):133-7, 2001)

WO 2005/00385 and Buxade et al., Immunity 23: 177-189, August 2005 both disclose a link between Mnks and the control of TNFα biosynthesis. The proposed mechanism is mediated by a regulatory AU-rich element (ARE) in the TNFα mRNA. Buxade et al. demonstrate proteins binding and controlling ARE function to be phosphorylated by Mnk1 and Mnk2. Specifically Mnk-mediated phosphorylation of the ARE-binding protein hnRNP A1 has been suggested to enhance translation of the TNFα mRNA.

TNFα is not the only cytokine regulated by an ARE. Functional AREs are also found in the transcripts of several interleukins, interferones and chemokines (Khabar, J Interf Cytokine Res 25: 1-10, 2005). The Mnk-mediated phosphorylation of ARE-binding proteins has thus the potential to control biosynthesis of cytokines in addition to that of TNFα.

Current evidence demonstrates Mnks as down stream targets of inflammatory signaling as well as mediators of the inflammatory response. Their involvement in the production of TNFα, RANTES, and potentially additional cytokines suggests inhibition of Mnks as strategy for anti-inflammatory therapeutic intervention.

Mnk1 and Mnk2 (including all splice forms) phosphorylate the translation factor elF4E on Serine 209. Mnk1/2 double knockout mice completely lack phosphorylation on Serine 209, indicating that Mnk kinase are the only kinases able to phosphorylate this site in vivo (Ueda et al., Mol Cell Biol. 2004; 24(15):6539-49). elF4E is overexpressed in a wide range of human malignancies, and high elF4E expression is frequently associated with more aggressive disease and poor prognosis. Furthermore, elF4E can act as an oncogene when assayed in standard assays for oncogenic activity (e.g. Ruggero et al., Nat Med. 2004 May;10(5):484-6). elF4E excerts its oncogenic activity by stimulating the translation of oncogenes such as c-myc and cyclinD1 (Culjkovic et al., J Cell Biol. 2006; 175(3):415-26), by increasing the expression of pro-survival factors such as MCP-1 (Wendel et al., Genes Dev. 2007; 21(24):3232-7) and by positively regulating pathways of drug resistance (Wendel et al., Nature 2004; 428(6980):332-7; Graff et el., Cancer Res. 2008; 68(3):631-4; De Benedetti and Graff, Oncogene 2004; 23(18):3189-99; Barnhart and Simon, J Clin Invest. 2007; 117(9):2385-8). Suppression of elF4E expression by antisense oligonucleotides has shown promise in preclinical experiments with human tumor cells (Graff et al., J Clin Invest. 2007; 117(9):2638-48). It has been shown that phosphorylation on Ser209 is strictly required for the oncogenic activity of elF4E in vitro and in vivo (Topisirovic et al., Cancer Res. 2004; 64(23):8639-42; Wendel et al., Genes Dev. 2007; 21(24):3232-7). Thus, inhibition of Mnk1 and Mnk2 is expected to have beneficial effects in human malignancies.

Inhibitors of Mnk (referred to as CGP57380 and CGP052088) have been described (cf. Mol. Cell. Biol. 21, 5500, 2001; Mol Cell Biol Res Comm 3, 205, 2000; Genomics 69, 63, 2000). CGP052088 is a staurosporine derivative having an IC₅₀ of 70 nM for inhibition of *in vitro* kinase activity of Mnk1. CGP57380 is a low molecular weight selective, non-cytotoxic inhibitor of Mnk2 (Mnk2a or Mnk2b) or of Mnk1: The addition of CGP57380 to cell culture cells, transfected with Mnk2 (Mnk2a or Mnk2b) or Mnk1 showed a strong reduction of phosphorylated eIF4E.

Further inhibitors of Mnk have been described. See for example Applicants patent applications WO 06/066937, describing pyrazolopyrimidine compounds, WO 06/136402 describing certain thienopyrimidine compounds, WO 07/115822 describing further thienopyrimidine compounds with modified core ring, and WO 08/006547 describing pyrrolopyrimidines as inhibitors of Mnk kinases.

The problem underlying the present invention is to provide potent and selective Mnk1 and/or Mnk2 inhibitors which may effectively and safely be used for the treatment of metabolic diseases, inflammatory diseases, cancer, neurodegenerative diseases and their consecutive complication and disorders.

It has now been surprisingly found that certain thienopyrimidine compounds are potent inhibitors of the kinase enzymes Mnk1 and/or Mnk2 and/or variants thereof and as such may be useful in the prophylaxis and/or therapy of diseases which can be influenced by the inhibition of the kinase activity of Mnk1 and/or Mnk2 (Mnk2a or Mnk2b) and/or variants thereof.

In contrast to the thienopyrimidine compounds known in the art, for example, the compounds disclosed in the Applicants patent applications WO 06/136402 and WO 2007/115822, the thienopyrimidine compounds of the present invention provide several advantages, namely, enhanced solubility, the possibility to form stable salts, improved metabolic stability, enhanced or retained activity in biochemical or cellular Mnk activity assays and enhanced or retained selectivity against other kinases.

The thienopyrimidine compounds disclosed in WO 06/136402 and WO 07/115822 exhibit high activity in Mnk enzyme assays and extremely high selectivity, however they show a very low solubility and are in most cases metabolic unstable resulting in undesired pharmacokinetic properties.

It has been surprisingly found that by the introduction of a polar group at the R⁴-position in the compounds of general formula (I) below leads to surprising substantial metabolic stabilization, rendering the thienopyrimidines of the present invention useful for in vivo pharmacological applications.

Moreover, compounds described in this application also show improved solubility, have strong inhibitory potency in biochemical and cellular assays and are highly selective, resulting in overall greatly improved pharmacological properties.

If not specified otherwise, any alkyl moiety mentioned in this application may be straight-chained or branched.

Thienopyrimidine compounds of the present invention are compounds of the general formula (I): 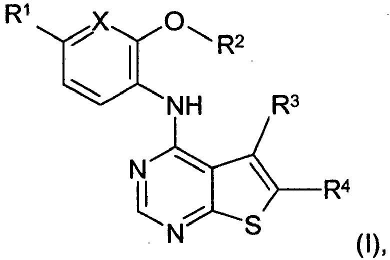 wherein
X is CH or N,
R¹ is a hydrogen or halogen atom, CN or CONH₂,
R² is a straight chain or branched C₁₋₆ alkyl group that is optionally substituted from position 2 onwards by one or two OH, C₁₋₄ alkoxy or NH₂, or that is substituted in any position by one to three F, or by a CO₂H, CONH₂, CONH(C₁₋₃ alkyl), CON(C₁₋₃ alkyl)₂, CONRR', SO₂NH₂, SO₂NH(C₁₋₃ alkyl) or SO₂N(C₁₋₃ alkyl)₂ group,
   wherein the rests R and R' together with the N atom to which they are attached form a 3-8 membered ring, wherein one CH₂ group may be replaced by O, S, SO, SO₂ or N, and wherein any carbon atom other than one attached to the nitrogen atom, may be substituted by OH, F C₁₋₃ alkyl or NH₂;
   a C₃₋₈ cycloalkyl group that is optionally substituted by one or two OH, C₁₋₄ alkoxy or NH₂ on any carbon atom other than one attached to the oxygen atom; or by F, CO₂H, CONH₂, CONH(C₁₋₃ alkyl), CON(C₁₋₃ alkyl)₂, SO₂NH₂, SO₂NH(C₁₋₃ alkyl), SO₂N(C₁₋₃ alkyl)₂, SO₂(C₁₋₃ alkyl), (CH₂)ₘOR⁵; (CH₂)ₘN(R⁵)₂,
   wherein the NH₂ groups mentioned above as substituent for the alkyl and cycloalkyl groups may optionally be independently substituted by C₁₋₃ alkyl, -(CH₂)m-OH, -(CH₂)ₘ-OCH₃, -(CH₂)ₘ-CN, -(CH₂)ₘ-F, SO₂(C₁₋₃ alkyl), CO₂(C₁₋₄ alkyl) or CO(C₁₋₃ alkyl);
   or a heterocyclyl system selected from any one of the following formulae: 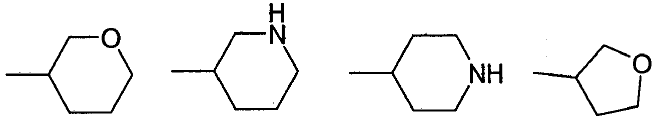 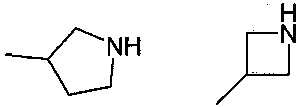 optionally substituted by one or more of R⁶,
   wherein the hydrogen atom of the NH group may optionally be replaced by C₁₋₃ alkyl, SO₂(C₁₋₃ alkyl), CO₂(C₁₋₄ alkyl) or CO(C₁₋₃ alkyl);
R³ is a C₁₋₂ alkyl group;
R⁴ is F, Cl, Br, I or CN;
R⁵ is selected from H and C₁₋₄ alkyl;
R⁶ is selected from OH, OR⁵ and N(R⁵)₂ on any carbon atom other than one attached to O or N; F; CO₂H; CON(R⁵)₂; SO₂N(R⁵)₂; SO₂R⁵; (CH₂)ₘOR⁵; (CH₂)ₘN(R⁵)₂; and
m is 1, 2 or 3;
or a tautomer, enantiomer, diastereomer or salt thereof.

Preferred compounds of formula (I) are those, wherein

X is CH or N,

R¹ is a hydrogen or fluorine atom or a CONH₂ group,
R² is a straight chain or branched C₁₋₄ alkyl group that is optionally substituted from position 2 onwards by NH₂; or a straight chain or branched C₁₋₄ alkyl group that is substituted in any position by one to three F; or a straight chain or branched C₁₋₄ alkyl group that is substituted in any position by CONH₂, CONH(C₁₋₃ alkyl), CON(C₁₋₃ alkyl)₂ or CONRR', wherein the rests R and R' together with the N atom to which they are attached form a 3-8 membered ring, wherein one CH₂ group may be replaced by O or N, and wherein any carbon atom may be substituted by OH, F or NH₂;
   a C₅₋₇ cycloalkyl group that is optionally substituted by one or two OH, C₁₋₄ alkoxy or NH₂ on any carbon atom other than one attached to the oxygen atom,
   wherein the NH₂ groups mentioned above as substituent for the alkyl and cycloalkyl groups may optionally be independently substituted by C₁₋₃ alkyl, -(CH₂)ₘ-OH, -(CH₂)ₘ-OCH₃, -(CH₂)ₘ-CN, -(CH₂)ₘ-F, SO₂(C₁₋₃ alkyl), CO₂(C₁₋₄ alkyl) or CO(C₁₋₃ alkyl);
   or heterocyclyl systems selected from any one of the following formulae: 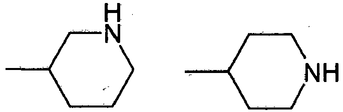 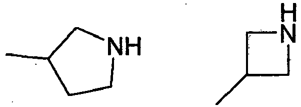 wherein the hydrogen atom of the NH group may optionally be replaced by SO₂(C₁₋₃ alkyl) or CO₂(C₁₋₄ alkyl);
R³ is a C₁₋₂ alkyl group;
R⁴ is F, Cl, Br or CN; and
m is 1, 2 or 3;
or a tautomer, enantiomer, diastereomer or salt thereof.

More preferred compounds of formula (I) are those, wherein

X is CH or N,

R¹ is a hydrogen or fluorine atom or a CONH₂ group,
R² is a straight chain or branched C₁₋₄ alkyl group that is optionally substituted from position 2 onwards by NH₂, or that is substituted in any position by one to three F;
   a cyclohexyl group that is optionally substituted by a OH, C₁₋₃ alkoxy or NH₂ on any carbon atom other than one attached to the oxygen atom,
   wherein the NH₂ groups mentioned above as substituent for the alkyl and cycloalkyl groups may optionally be independently substituted by C₁₋₃ alkyl, SO₂(C₁₋₃ alkyl), CO₂(C₁₋₄ alkyl) or CO(C₁₋₃ alkyl);
   or heterocyclyl systems selected from any one of the following formulae: 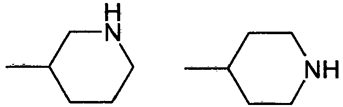 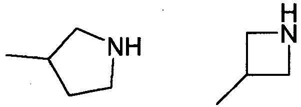 wherein the hydrogen atom of the NH group may optionally be replaced by SO₂(C₁₋₃ alkyl) or CO₂(C₁₋₄ alkyl);
R³ is a C₁₋₂ alkyl group and
R⁴ is F, Cl or Br,
or a tautomer, enantiomer, diastereomer or salt thereof.

A preferred subgroup concerns those compounds of formula (I), wherein
X and R¹, R² and R⁴ are as defined above and
R³ is CH₃,
or a tautomer, enantiomer, diastereomer or salt thereof.

Another preferred subgroup concerns those compounds of formula (I), wherein
R² to R⁴ are as defined above,
X is CH and
R¹ is F or CONH₂,
or a tautomer, enantiomer, diastereomer or salt thereof.

A third preferred subgroup concerns those compounds of formula (I), wherein
R² to R⁴ are as defined above,
X is N and
R¹ is H,
or a tautomer, enantiomer, diastereomer or salt thereof.

A fourth preferred subgroup concerns those compounds of formula (I), wherein
X and R¹ to R³ are as defined above and
R⁴ is F, Cl or Br,
or a tautomer, enantiomer, diastereomer or salt thereof,
even more preferred are those compounds of formula (I), wherein
X and R¹ to R³ are as defined above and
R⁴ is Cl or Br,
or a tautomer, enantiomer, diastereomer or salt thereof.

Particularly preferred compounds are:
a) (trans-3-[2-(6-Chloro-5-methyl-thieno[2,3-*d*]pyrimidin-4-ylamino)-5-fluoro-phenoxy]-cyclohexanol,
b) (6-Chloro-5-methyl-thieno[2,3-*d*]pyrimidin-4-yl)-[2-(trans-4-methylamino-cyclohexyloxy)-pyridin-3-yl]-amine,
c) (6-Chloro-5-methyl-thieno[2,3-*d*]pyrimidin-4-yl)-[4-fluoro-2-(2-fluoro-1-fluoromethyl-ethoxy)-phenyl]-amine,
d) (6-Chloro-5-methyl-thieno[2,3-*d*]pyrimidin-4-yf)-[4-fluoro-2-((R)-piperidin-3-yloxy)-phenyl]-amine,
e) 6-Chloro-5-methyl-thieno[2,3-*d*]pyrimidin-4-yl)-[4-fluoro-2-((R)-1-methanesulfonyl-piperidin-3-yloxy)-phenyl]-amine,
f) [2-(2-Amino-1-methyl-ethoxy)-4-fluoro-phenyl]-(6-bromo-5-methyl-thieno[2,3-*d*]pyrimidin-4-yl)-amine,
g) *N*-{2-[2-(6-Bromo-5-methyl-thieno[2,3-*d*]pyrimidin-4-ylamino)-5-fluoro-phenoxy]-propyl}-methanesulfonamide,
h) (6-Bromo-5-methyl-thieno[2,3-*d*]pyrimidin-4-yl)-(4-fluoro-2-(tetrahydro-pyran-4-yloxy)-phenyl]-amine,
i) [2-(2-Amino-1-methyl-ethoxy)-4-fluoro-phenyl]-(6-chloro-5-methyl-thieno[2,3-*d*]pyrimidin-4-yl)-amine,
j) *N*-{2-[2-(6-Chloro-5-methyl-thieno[2,3-*d*]pyrimidin-4-ylamino)-5-fluoro-phenoxy]-propyl}-acetamide,
k) *N*-{2-[2-(6-Chloro-5-methyl-thieno[2,3-*d*]pyrimidin-4-ylamino)-5-fluoro-phenoxy]-propyl}-methanesulfonamide,
l) 4-(6-Chloro-5-methyl-thieno[2,3-*d*]pyrimidin-4-ylamino)-3-ethoxy-benzamide,
m) 4-(6-Bromo-5-methyl-thieno[2,3-*d*]pyrimidin-4-ylamino)-3-ethoxy-benzamide and
n) (6-Bromo-5-methyl-thieno[2,3-*d*]pyrimidin-4-yl)-(4-fluoro-2-isopropoxy-phenyl)-amine,
or a pharmaceutically acceptable salt thereof.

Typical methods of preparing the compounds of the invention are described below in the experimental section.

The potent inhibitory effect of the compounds of the invention may be determined by in vitro enzyme assays as described in the Examples in more detail.

### General Synthesis:

The compounds of the present invention can be synthesized according to the following schemes:

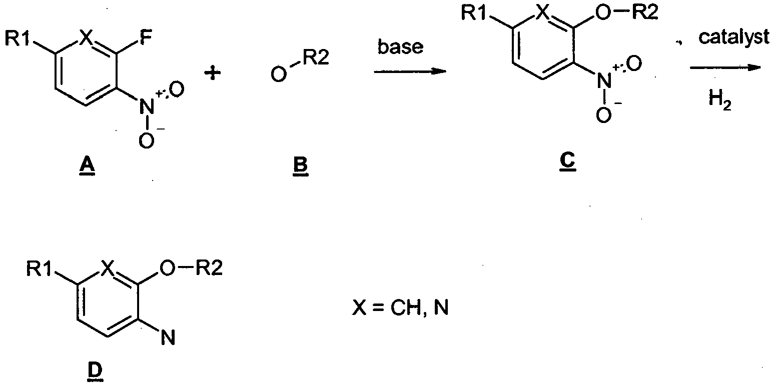

Compounds of the general formula **C** can be synthesized by reaction of a compound **A** with the deprotonated alcohol **B** in appropriate solvents such as THF or DMF at a temperature between 0 °C and 150 °C. The deprotonated form of **B** can be obtained by deprotonation with a base such as sodium hydride or lithium hexamethyldisilazane at a preferred temperature of 0 °C. Hydrogenation of compound **C** in order to obtain a compound of the general formula **D** can be achieved by reacting **C** in the presence of hydrogen and a catalyst such as palladium or Raney nickel. The hydrogen can be introduced as a gas or generated from a hydrogen source such as ammonium formate.

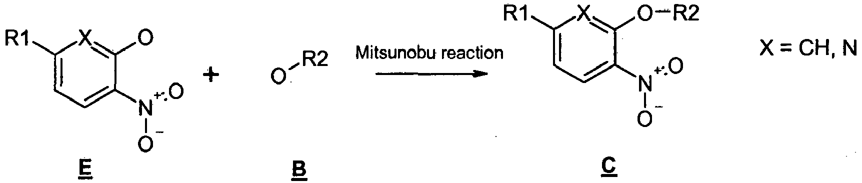

Compounds of the general formula **C** can be also obtained by Mitsunobu reaction of a compound with the general formula **E** with an alcohol **B** in the presence of triphenylphosphine and an dialkylazodicarboxylate such as diethylazodicarboxylate, diisopropylazodicarboxylate or di-tert.butylazodiacarboxylate in a solvent such as THF at temperatures between -10 °C and 80 °C, preferrably between 0 °C and 30 °C. 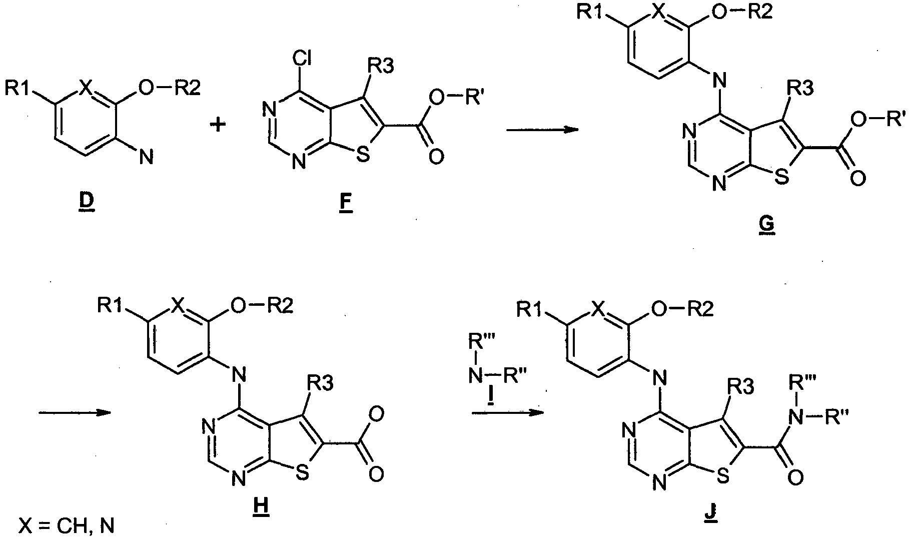 A compound of the formula **G** can be synthesized by reaction of compound **D** with **F** preferably in the presence of an acid such as p-toluene sulfonic acid or hydrochloric acid in solvents such as dioxane at temperatures between 10 °C and 150 °C. Synthesis of a compound with the general formula **H** can be achieved by reaction of compound **G** with a base such as sodium hydroxide or lithium hydroxide in solvents such as methanol, ethanol, THF and water or mixtures thereof, preferably in ethanol/THF or THF/water at temperatures between 10 °C and 100 °C. A compound of the general formula **J** can be obtained by reaction of compound **H** with amines of the general formula **I** using amide coupling procedures employing reagents such as TBTU, HATU or EDC/ N-Hydroxysuccinimide in the presence or absence of bases such as diisopropylethylamine in solvents such as DMF or THF at temperatures between 0 °C and 120 °C preferably between 0 °C and 30 °C.

6-Halo-thieno[2,3-*d*]pyrimidines can be prepared by halogenation of thieno[2,3-*d*]pyrimidines with N-Halo-succinimide in acetic acid at elevated temperatures preferably between 60 °C and 90 °C.

6-Cynao-thieno[2,3-*d*]pyrimidines can be prepared by dehydration of the corresponding thieno[2,3-*d*]pyrimidin-6-carboxamides with dehydrating agents such as acetic anhydride, trifluoracetic anhydride, phosphorus oxychloride in pyridine, pyridine/dichloromethane at low temperatures preferably between -10 °C and 10 °C.

Pharmaceutically acceptable salts of the compounds of the invention of formula (1) can be formed with numerous organic and inorganic acids and bases. Exemplary acid addition salts including acetate, adipate, alginate, ascorbate, aspartate, benzoate, benzenesulfonate, bisulfate, borate, butyrate, citrate, camphorate, camphersulfonate, cyclopentanepropionate, digluconate, dodecyl sulfate, ethane sulfonate, fumarate, glucoheptanoate, glycerophosphate, hemisulfate, heptanoate, hexanoate, hydrochloride, hydrobromide, hydroiodide, 2-hydroxyethane sulfonate, lactate, maleate, methane sulfonate, 2-naphthalene sulfonate, nicotinate, nitrate, oxalate, pamoate, pectinate, persulfate, 3-phenyl sulfonate, 3-phenylpropionate, phosphate, picrate, pivalate, propionate, salicylate, succinate, sulfate, sulfonate, tartrate, thiocyanate, toluene sulfonate such as tosylate, undecanoate, or the like.

Basic nitrogen-containing moieties can be quaternized with such agents as lower alkyl halides, such as methyl, ethyl, propyl, and butyl chloride, bromide and iodide; dialkyl sulfates like dimethyl, diethyl, dibutyl, and diamyl sulfates, long-chain alkyl halides such as decyl, lauryl, myristyl and stearyl chloride, bromide and iodide, or aralkyl halides like benzyl and phenethyl bromides, or others. Water soluble or dispersible products are thereby obtained.

Pharmaceutically acceptable basic addition salts include but are not limited to cations based on the alkaline and alkaline earth metals such as sodium, lithium, potassium, calcium, magnesium, aluminum salts and the like, as well as non toxic ammonium quarternary ammonium, and amine cations, including but not limited to ammonium, tetramethylammonium, tetraethylammonium, methylamine, dimethylamine, trimethylamine, triethylamine, ethylamine and the like. Other representative amines useful for the formation of base addition salts include benzazethine, dicyclohexyl amine, hydrabine, N-methyl-D-glucamine, N-methyl-D-glucamide, t-butyl amine, diethylamine, ethylendiamine, ethanolamine, diethanolamine, piperazine and the like and salts with amino acids such as arginine, lysine, or the like.

Unless specifically indicated, throughout the specification and the appended claims, a given chemical formula or name shall encompass tautomers and all stereo, optical and geometrical isomers (e.g. enantiomers, diastereomers, E/Z isomers etc...) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates of the free compounds or solvates of a salt of the compound.

As used herein the term "metabolite" refers to (i) a product of metabolism, including intermediate and products, (ii) any substance involved in metabolism (either as a product of metabolism or as necessary for metabolism), or (iii) any substance produced or used during metabolism. In particular it refers to the end product that remains after metabolism.

As used herein the term "prodrug" refers to (i) an inactive form of a drug that exerts its effects after metabolic processes within the body convert it to a usable or active form, or (ii) a substance that gives rise to a pharmacologically active metabolite, although not itself active (i.e. an inactive precursor).

The terms "prodrug" or "prodrug derivative" mean a covalently-bonded derivative or carrier of the parent compound or active drug substance which undergoes at least some biotransformation prior to exhibiting its pharmacological effect(s). In general, such prodrugs have metabolically cleavable groups and are rapidly transformed in vivo to yield the parent compound, for example, by hydrolysis in blood, and generally include esters and amide analogs of the parent compounds. The prodrug is formulated with the objectives of improved chemical stability, improved patient acceptance and compliance, improved bioavailability, prolonged duration of action, improved organ selectivity, improved formulation (e.g., increased hydrosolubility), and/or decreased side effects (e.g., toxicity). In general, prodrugs themselves have weak or no biological activity and are stable under ordinary conditions. Prodrugs can be readily prepared from the parent compounds using methods known in the art, such as those described in A Textbook of Drug Design and Development, Krogsgaard-Larsen and H. Bundgaard (eds.), Gordon & Breach, 1991, particularly Chapter 5: "Design and Applications of Prodrugs"; Design of Prodrugs, H. Bundgaard (ed.), Elsevier, 1985; Prodrugs: Topical and Ocular Drug Delivery, K.B. Sloan (ed.), Marcel Dekker, 1998; Methods in Enzymology, K. Widder et al. (eds.), Vol. 42, Academic Press, 1985, particularly pp. 309-396; Burger's Medicinal Chemistry and Drug Discovery, 5th Ed., M. Wolff (ed.), John Wiley & Sons, 1995, particularly Vol. 1 and pp. 172-178 and pp. 949-982; Pro-Drugs as Novel Delivery Systems, T. Higuchi and V. Stella (eds.), Am. Chem. Soc., 1975; Bioreversible Carriers in Drug Design, E.B. Roche (ed.), Elsevier, 1987.

The term "pharmaceutically acceptable prodrug" as used herein means a prodrug of a compound of the invention which is, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible.

As used herein the term "C₃₋₁₀ cycloalkyl" or "C₃₋₈ cycloalkyl" refers to mono- or polycyclic carbocyclic alkyl substituent or group having 3 to 10 or 3 to 8 ring atoms respectively, such as cyclopropyl, cyclobutyl, cyclopentyl, cyclohexyl, cycloheptyl, cyclobutenyl, cyclopentenyl, cyclopentadienyl, cyclohexenyl, cyclohexadienyl, cycloheptenyl, cycloheptadienyl, cycloheptatrienyl perhydrated naphthalene or indene, adamantyl or norbonanyl and the like.

The term "C₁₋₈ alkyl" as used herein alone or in combination with other terms such as in alkoxy refers to a C₁₋₈, preferably C₁₋₄ straight or branched alkyl/alkoxy group such as methyl, ethyl, propyl (iso-, n-), butyl (iso-, n-, sec-, tert-), pentyl, hexyl, methoxy, ethoxy, propoxy (iso-, n-), butoxy (iso-, n-, sec-, tert-), pentoxy, hexoxy.

The term "C₂₋₈ alkenyl" by itself or as part of another group refers to a straight or branched alkenyl group of 2 to 8 carbons, preferably 2 to 6 carbons, in the normal chain, which include one or more double bonds in the normal chain, such as vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl.

The term "heterocyclyl" refers to monocyclic saturated or unsaturated heterocyclyl groups with 1 to 4 hetero atoms selected from N, S and O, with the remainder of the ring atoms being carbon atoms and having preferably a total number of ring atoms of 3 to 10, such as morpholino, piperazinyl, piperidinyl, pyridyl, pyrimidinyl, thiazolyl, indolyl, imidazolyl, oxadiazolyl, tetrazolyl, pyrazinyl, triazolyl, thiophenyl or furanyl.

The term "heteroaryl" refers to a mono- or bicyclic aromatic group with 1 to 4 hetero atoms selected from N, S and O, with the remainder of the ring atoms being carbon atoms and having preferably a total number of ring atoms of 5 to 10. Examples without limitation of heteroaryl groups are such as benzofuranyl, furyl, thienyl, benzothienyl, thiazolyl, imidazolyl, oxazolyl, oxadiazolyl, thiadiazolyl, benzothiazolyl, triazolyl, tetrazolyl, isoxazolyl, isothiazolyl, pyrrolyl, pyranyl, tetrahydropyranyl, pyrazolyl, pyridyl, quinolinyl, isoquinolinyl, purinyl, carbazolyl, benzoxazolyl, benzamidazolyl, indolyl, isoindolyl, pyrazinyl, diazinyl, pyrazine, triazinyltriazine, tetrazinyl, tetrazolyl, benzothiophenyl, benzopyridyl and benzimidazolyl.

In a further aspect the present invention provides pharmaceutical compositions comprising a thienopyrimidine compound of the present invention and optionally a pharmaceutically acceptable carrier.

The pharmaceutical composition according to the present invention may further comprise an additional therapeutic agent. Particularly preferred are compositions, wherein the additional therapeutic agent is selected from antidiabetics like insulin, long and short acting insulin analogues, sulfonylureas, biguanides, DPP-IV inhibitors, SGLT2 inhibitors, 11ß-HSD inhibitors, glucokinase activators, AMPK activators, Glp-1 receptor agonists, GIP receptor agonists, DGAT inhibitors, PPARgamma agonists, PPARdelta agonists, and other antidiabetics derived from thiazolidinediones, lipid lowering agents such as statines, fibrates, ion exchange resins nicotinic acid derivatives, or HMG-CoA reductase inhibitors, cardiovascular therapeutics such as nitrates, antihypertensiva such as β-blockers, ACE inhibitors, Ca-channel blockers, angiotensin II receptor antagonists, diuretics, thrombocyte aggregation inhibitors, or antineoplastic agents such as alkaloids, alkylating agents, antibiotics, or antimetabolites, or anti-obesity agents. Further preferred compositions are compositions wherein the additional therapeutic agent is selected from a histamine antagonist, a bradikinin antagonist, serotonin antagonist, leukotriene, an antiasthmatic, an NSAID, an antipyretic, a corticosteroid, an antibiotic, an analgetic, a uricosuric agent, chemotherapeutic agent, an anti gout agent, a bronchodilator, a cyclooxygenase-2 inhibitor, a steroid, a 5-lipoxygenase inhibitor, an immunosuppressive agent, a leukotriene antagonist, a cytostatic agent, an antineoplastic agent, a mTor inhibitor, a Tyrosine kinase inhibitor, antibodies or fragments thereof against cytokines and soluble parts (fragments) of cytokine receptors.

More particularly preferred are compounds such as human NPH insulin, human lente or ultralente insulin, insulin Lispro, insulin Aspart, insulin Glulisine, insulin detemir or insulin Glargine, metformin, phenformin, acarbose, miglitol, voglibose, pioglitazone, rosiglizatone, rivoglitazone, aleglitazar, alogliptin, saxagliptin, sitagliptin, vildagliptin, exenatide, liraglutide, albiglutide, pramlintide, carbutamide, chlorpropamide, glibenclamide (glyburide), gliclazide, glimepiride, glipizide, gliquidone, tolazamide, tolbutamide, atenolol, bisoprolol, metoprolol, esmolol, celiprolol, talinolol, oxprenolol, pindolol, propanolol, bupropanolol, penbutolol, mepindolol, sotalol, certeolol, nadolol, carvedilol, nifedipin, nitrendipin, amlodipin, nicardipin, nisoldipin, diltiazem, enalapril, verapamil, gallopamil, quinapril, captopril, lisinopril, benazepril, ramipril, peridopril, fosinopril, trandolapril, irbesatan, losartan, valsartan, telmisartan, eprosartan, olmesartan, hydrochlorothiazide, piretanid, chlorotalidone, mefruside, furosemide, bendroflumethiazid, triamterene, dehydralazine, acetylsalicylic acid, tirofiban-HCl, dipyramidol, triclopidin, iloprost-trometanol, eptifibatide, clopidogrel, piratecam, abciximab, trapidil, simvastatine, bezafibrate, fenofibrate, gemfibrozil, etofyllin, clofibrate, etofibrate, fluvastatine, lovastatine, pravastatin, colestyramide, colestipol-HCl, xantinol nicotinat, inositol nicotinat, acipimox, nebivolol, glycerolnitrate, isosorbide mononitrate, isosorbide dinitrate, pentaerythrityl tetranitrate, indapamide, cilazepril, urapidil, eprosartan, nilvadipin, metoprolol, doxazosin, molsidormin, moxaverin, acebutolol, prazosine, trapidil, clonidine, vinca alkaloids and analogues such as vinblastin, vincristin, vindesin, vinorelbin, podophyllotoxine derivatives, etoposid, teniposid, alkylating agents, nitroso ureas, N-lost analogues, cycloplonphamid, estamustin, melphalan, ifosfamid, mitoxantron, idarubicin, doxorubicin, bleomycin, mitomycin, dactinomycin, daptomycin, docetaxel, paclitaxel, carboplatin, cisplatin, oxaliplatin, BBR3464, satraplatin, busulfan, treosulfan, procarbazine, dacarbazine, temozolomide, chlorambucil, chlormethine, cyclophosphamide, ifosfamide, melphalan, bendamustine, uramustine, ThioTEPA, camptothecin, topotecan, irinotecan, rubitecan, etoposide, teniposide, cetuximab, panitumumab, trastuzumab, rituximab, tositumomab, alemtuzumab, bevacizumab, gemtuzumab, aminolevulinic acid, methyl aminolevulinate, porfimer sodium, verteporfin, axitinib, bosutinib, cediranib, dasatinib, erlotinib, gefitinib, imatinib, lapatinib, lestaurtinib, nilotinib, semaxanib, sorafenib, sunitinib, vandetanib, retinoids (alitretinoin, tretinoin), altretamine, amsacrine, anagrelide, arsenic trioxide, asparaginase (pegaspargase), bexarotene, bortezomib, denileukin diftitox, estramustine, ixabepilone, masoprocol, mitotane, testolactone, tipifarnib, abetimus, deforolimus, everolimus, gusperimus, pimecrolimus, sirolimus, tacrolimus, temsirolimus, antimetabolites such as cytarabin, fluorouracil, fluoroarabin, gemcitabin, tioguanin, capecitabin, combinations such as adriamycin/daunorubicin, cytosine arabinosid/cytarabine, 4-HC, or other phosphamides.

Other particularly preferred compounds are compounds such as clemastine, diphenhydramine, dimenhydrinate, promethazine, cetirizine, astemizole, levocabastine, loratidine, terfenadine, acetylsalicylic acid, sodoum salicylate, salsalate, diflunisal, salicylsalicylic acid, mesalazine, sulfasalazine, osalazine, acetaminophen, indomethacin, sulindac, etodolac, tolmetin, ketorolac, bethamethason, budesonide, chromoglycinic acid, dimeticone, simeticone, domperidone, metoclopramid, acemetacine, oxaceprol, ibuprofen, naproxen, ketoprofen, flubriprofen, fenoprofen, oxaprozin, mefenamic acid, meclofenamic acid, pheylbutazone, oxyphenbutazone, azapropazone, nimesulide, metamizole, leflunamide, eforicoxib, lonazolac, misoprostol, paracetamol, aceclofenac, valdecoxib, parecoxib, celecoxib, propyphenazon, codein, oxapozin, dapson, prednisone, prednisolon, triamcinolone, dexibuprofen, dexamethasone, flunisolide, albuterol, salmeterol, terbutalin, theophylline, caffeine, naproxen, glucosamine sulfate, etanercept, ketoprofen, adalimumab, hyaluronic acid, indometacine, proglumetacine dimaleate, hydroxychloroquine, chloroquine, infliximab, etofenamate, auranofin, gold, [²²⁴Ra]radium chloride, tiaprofenic acid, dexketoprofen(trometamol), cloprednol, sodium aurothiomalate aurothioglucose, colchicine, allopurinol, probenecid, sulfinpyrazone, benzbromarone, carbamazepine, lornoxicam, fluorcortolon, diclofenac, efalizumab, idarubicin, doxorubicin, bleomycin, mitomycin, dactinomycin, daptomycin, cytarabin, fluorouracil, fluoroarabin, gemcitabin, tioguanin, capecitabin, adriamydin/daunorubicin, cytosine arabinosid/cytarabine, 4-HC, or other phosphamides, penicillamine, a hyaluronic acid preparation, arteparon, glucosamine, MTX, soluble fragments of the TNF-receptor (such as etanercept (Enbrel)) and antibodies against TNF (such as infliximab (Remicade), natalizumab (Tysabri) and adalimumab (Humira)).

It will be appreciated by the person of ordinary skill in the art that the compounds of the invention and the additional therapeutic agent may be formulated in one single dosage form, or may be present in separate dosage forms and may be either administered concomitantly (i.e. at the same time) or sequentially.

The pharmaceutical compositions of the present invention may be in any form suitable for the intended method of administration.

The compounds of the present invention may be administered orally, parenterally, such as bronchopulmonary, subcutaneously, intravenously, intramuscularly, intraperitoneally, intrathecally, transdermally, transmucosally, subdurally, locally or topically via iontopheresis, sublingually, by inhalation spray, aerosol or rectally and the like in dosage unit formulations optionally comprising conventional pharmaceutically acceptable excipients.

Excipients that may be used in the formulation of the pharmaceutical compositions of the present invention comprise carriers, vehicles, diluents, solvents such as monohydric alcohols such as ethanol, isopropanol and polyhydric alcohols such as glycols and edible oils such as soybean oil, coconut oil, olive oil, safflower oil cottonseed oil, oily esters such as ethyl oleate, isopropyl myristate; binders, adjuvants, solubilizers, thickening agents, stabilizers, disintergrants, glidants, lubricating agents, buffering agents, emulsifiers, wetting agents, suspending agents, sweetening agents, colorants, flavors, coating agents, preservatives, antioxidants, processing agents, drug delivery modifiers and enhancers such as calcium phosphate, magnesium state, talc, monosaccharides, disaccharides, starch, gelatine, cellulose, methylcellulose, sodium carboxymethyl cellulose, dextrose, hydroxypropyl-ß-cyclodextrin, polyvinylpyrrolidone, low melting waxes, ion exchange resins.

Other suitable pharmaceutically acceptable excipients are described in Remington's Pharmaceutical Sciences, 15th Ed., Mack Publishing Co., New Jersey (1991).

Dosage forms for oral administration include tablets, capsules, lozenges, pills, wafers, granules, oral liquids such as syrups, suspensions, solutions, emulsions, powder for reconstitution.

Dosage forms for parenteral administration include aqueous or olageous solutions or emulsions for infusion, aqueous or olageous solutions, suspensions or emulsions for injection pre-filled syringes, and/or powders for reconstitution.

Dosage forms for local/topical administration comprise insulations, aerosols, metered aerosols, transdermal therapeutic systems, medicated patches, rectal suppositories, and/or ovula.

The amount of the compound of the present invention that may be combined with the excipients to formulate a single dosage form will vary upon the host treated and the particular mode of administration.

The pharmaceutical compositions of the invention can be produced in a manner known per se to the skilled person as described, for example, in Remington's Pharmaceutical Sciences, 15th Ed., Mack Publishing Co., New Jersey (1991).

In a further aspect of the invention the use of a thienopyrimidine compound of the present invention for the production of a pharmaceutical composition for inhibiting the activity of the kinase activity of Mnk1 or Mnk2 (Mnk2a, Mnk2b) or further variants thereof is provided, in particular for the prophylaxis or therapy of metabolic diseases, hematopoietic disorders, cancer and their consecutive complications and disorders. Whereby the prophylaxis and therapy of metabolic diseases of the carbohydrate and/or lipid metabolism is preferred.

Diseases of the invention that are influenced by the inhibition of the kinase activity of Mnk1 and/or Mnk2 (Mnk2a or Mnk2b) and/or further variants thereof include diseases related to the regulation of metabolic diseases, such as obesity, eating disorders, cachexia, diabetes mellitus, metabolic syndrome, hypertension, coronary heart diseases, hypercholesterolemia, dyslipidemia, osteoarthritis, biliary stones and/or sleep apnea and diseases related to reactive oxygen compounds (ROS defense) such as diabetes mellitus, neurodegenerative diseases and cancer.

The pharmaceutical compositions of the invention are particularly useful for prophylaxis and treatment of obesity, diabetes mellitus and other metabolic diseases of the carbohydrate and lipid metabolism as stated above, in particular diabetes mellitus and obesity.

Thus in a more preferred embodiment of this invention the use of a thienopyrimidine compound for the production of a pharmaceutical composition for the prophylaxis or therapy of metabolic diseases is provided.

In yet a further aspect of the invention the use of a thienopyrimidine compound of the invention for the production of a pharmaceutical composition for treating or preventing a cytokine mediated disorder such as an inflammatory disease is provided.

The pharmaceutical compositions of the invention are thus useful for the prophylaxis or therapy of inflammatory diseases, in particular chronic or acute inflammation, chronic inflammatory arthritis, rheumatoid arthritis, psoriatic arthritis, osteoarthritis, juvenile rheumatoid arthritis, gouty arthritis; psoriasis, erythrodermic psoriasis, pustular psoriasis, inflammatory bowel disease, Crohn's disease and related conditions, ulcerative colitis, colitis, diverticulitis, nephritis, urethritis, salpingitis, oophoritis, endomyometritis, spondylitis, systemic lupus erythematosus and related disorders, multiple sclerosis, asthma, meningitis, myelitis, encephalomyelitis, encephalitis, phlebitis, thrombophlebitis, chronic obstructive disease (COPD), inflammatory lung disease, allergic rhinitis, endocarditis, osteomyelitis, rheumatic fever, rheumatic pericarditis, rheumatic endocarditis, rheumatic myocarditis, rheumatic mitral valve disease, rheumatic aortic valve disease, prostatitis, prostatocystitis, spondoarthropathies ankylosing spondylitis, synovitis, tenosynovotis, myositis, pharyngitis, polymyalgia rheumatica, shoulder tendonitis or bursitis, gout, pseudo gout, vasculitides, inflammatory diseases of the thyroid selected from granulomatous thyroiditis, lymphocytic thyroiditis, invasive fibrous thyroiditis, acute thyroiditis; Hashimoto's thyroiditis, Kawasaki's disease, Raynaud's phenomenon, Sjogren's syndrome, neuroinflammatory disease, sepsis, conjubctivitis, keratitis, iridocyclitis, optic neuritis, otitis, lymphoadenitis, nasopaharingitis, sinusitis, pharyngitis, tonsillitis, laryngitis, epiglottitis, bronchitis, pneumonitis, stomatitis, gingivitis, oesophagitis, gastritis, peritonitis, hepatitis, cholelithiasis, cholecystitis, glomerulonephritis, goodpasture's disease, crescentic glomerulonephritis, pancreatitis, dermatitis, endomyometritis, myometritis, metritis, cervicitis, endocervicitis, exocervicitis, parametritis, tuberculosis, vaginitis, vulvitis, silicosis, sarcoidosis, pneumoconiosis, inflammatory polyarthropathies, psoriatric arthropathies, intestinal fibrosis, bronchiectasis and enteropathic arthropathies.

As already stated above, the compositions of the present invention are particularly useful for treating or preventing a disease selected from chronic or acute inflammation, chronic inflammatory arthritis, rheumatoid arthritis, psoriasis, COPD, inflammatory bowel disease, septic shock, Crohn's disease, ulcerative colitis, multiple sclerosis and asthma.

Thus, in a more preferred embodiment of this invention the use of a thienopyrimidine compound for the production of a pharmaceutical composition for the prophylaxis or therapy of inflammatory diseases selected from chronic or acute inflammation, chronic inflammatory arthritis, rheumatoid arthritis, psoriasis, COPD, inflammatory bowel disease, septic shock Crohn's disease, ulcerative colitis, multiple sclerosis and asthma is provided.

In yet a further aspect of the invention the use of a thienopyrimidine compound of the invention for the production of a pharmaceutical composition for treating or preventing cancer, viral diseases or neurodegenerative diseases is provided.

For the purpose of the present invention, a therapeutically effective dosage will generally be from about 1 to 2000 mg/day, preferably from about 10 to about 1000 mg/day, and most preferably from about 10 to about 500 mg/day, which may be administered in one or multiple doses.

It will be appreciated, however, that specific dose level of the compounds of the invention for any particular patient will depend on a variety of factors such as age, sex, body weight, general health condition, diet, individual response of the patient to be treated time of administration, severity of the disease to be treated, the activity of particular compound applied, dosage form, mode of application and concomitant medication. The therapeutically effective amount for a given situation will readily be determined by routine experimentation and is within the skills and judgment of the ordinary clinician or physician.

### Kinase Fluorescence Polarization Assays

**Assay principle:** Inhibitory potency of compounds against Mnk1, Mnk2a and other kinases was assessed with assays based on a format known to those skilled in the art as the indirect (competitive) fluorescence polarization. The assay detection system comprises a small fluorophore-labeled phospho-peptide (termed ligand) bound to a phospho-specific antibody. The product generated by the kinase reaction competes with the ligand for antibody binding. Based on the larger molecular volume of the bound ligand, which results in a lower rotation rate in solution, its emitted light has a higher degree of polarization than the one from the free ligand.

### Description of the specific homogenous kinase assay

### Example 2a. Mnk1 and Mnk2a in vitro kinase assay

As a source of enzyme, human Mnk1 and human Mnk2a were expressed as GST fusion proteins in *E. coli*, purified to >80% homogeneity by glutathione affinity chromatography and activated *in vitro* with pre-activated ERK2. In brief, the open reading frames of human Mnk1 and Mnk2a were amplified from cDNA using the forward/reverse primer pairs
SEQ ID NO: 1 5'TTTAGGATCCGTATCTTCTCAAAAGTTGG /
SEQ ID NO: 2 5' CTGGGTCGACTCAGAGTGCTGTGGGCGG and
SEQ ID NO: 3 5'ACAGGGATCCGTGCAGAAGAAACCAGCC /
SEQ ID NO: 4 5'GATGGTCGACTCAGGCGTGGTCTCCCACC
(utilized restriction sites underlined), respectively, and cloned into the BamHI and SalI sites of the vector pGEX-4T1 (Amersham, Sweden, cat. no. 27-4580-01). These constructs allow prokaryotic expression of Mnk1 or Mnk2a as fusion protein with a N-terminal glutathione S-transferase (GST) tag, referred to as GST-Mnk1 or GST-Mnk2a. The following expression and purification procedure was identical for GST-Mnk1 and GST-Mnk2a, referring in general to GST-Mnk, when not distinguishing between the two isoforms. Expression of GST-Mnk was in *E*. *coli* BL21 (Merck Biosciences, Germany, cat. no. 69449). Cells were grown in LB-Bouillon (Merck, Germany, cat. no. 1.10285) supplemented with 100 µg/ml ampicillin (Sigma, Germany, cat. no. A9518) at 37°C. When the culture had reached a density corresponding to an A₆₀₀ of 0.8, an equal volume of ice cold LB/ampicillin was added, the culture transferred to 25°C and induced for 4 h with 1 mM isopropyl thiogalactoside (IPTG, Roth, Germany, cat. no. 2316.4). Cells harvested by centrifugation were resuspended in 10 ml lysis buffer (50 mM tris(hydroxymethyl)aminomethane hydrochloride (Tris/HCl, Sigma, Germany, cat. no. T5941) pH 7.5, 300 mM sodium chloride (NaCl, Sigma, Germany, cat. no. S7653), 5% (w/v) glycerol (Sigma, Germany, cat. no. G5516), 3 mM DTT dithiotreitol (DTT, Sigma, Germany, cat. no. D9779)) per gram wet weight cell pellet. Lysates were prepared by disruption of cells with a sonifier and subsequent clearing by centrifugation at 38000 g for 45 min at 4°C.

The lysate was applied to a GSTPrep FF 16/10 column (Amersham, Sweden, cat. no. 17-5234-01) equilibrated with lysis buffer. Removal of unbound material was with 3 column volumes (CV) lysis buffer. Elution was with 2 CV of elution buffer (50 mM Tris/HCl pH 7.5, 300 mM NaCl, 5% (w/v) glycerol, 20 mM glutathione (Sigma, Germany, cat. no. G4251)). Peak fractions were pooled and the protein transferred into storage buffer (50 mM Tris/HCl pH 7.5, 200 mM NaCl, 0.1 mM ethylene glycol-bis(2-aminoethylether)-N,N,N',N'-tetraacetic acid (EGTA, Aldrich, Germany, cat. no. 23,453-2), 1 mM DTT, 10% (w/v) glycerol, 0.5 M sucrose (Sigma, Germany, cat. no. S0389) by gel filtration on a PD10 desalting column (Amersham, Sweden, cat. no. 17-0851-01). Aliquots were shock frozen in liquid nitrogen and stored at -80°C.

Activation of Mnk1 and Mnk2a was at a concentration of 2.5 µM of either purified GST-Mnk1 or GST-Mnk2a by incubation with 150 nM pre-activated NHis-ERK2 (see ERK2 assay for preparation) and 50 µM adenosine triphosphate (ATP, Sigma, cat. no. A2699) in a buffer comprising 20 mM N-(2-hydroxyethyl) piperazine-N'-(2-ethanesulfonic acid) (HEPES, Fluka, Germany, cat. no 54459)/potassium hydroxide (KOH, Roth, Germany, cat. no 6751.1) pH 7.4, 10 mM magnesium chloride (MgCl₂, Sigma, Germany, cat. no. M2670), 0.25 mM DTT, 0.05% (w/v) polyoxyethylene 20 stearylether (Brij 78, Sigma, Germany, cat. no. P4019) (HMDB buffer) for 45 min at 30°C. After the incubation, the preparation was aliquoted into single-use samples, shock frozen in liquid nitrogen, stored at -80°C and utilized for Mnk1 or Mnk2a kinase assays as detailed below. The presence of activating kinase has been tested to not interfere with the Mnk activity assay.
SUBSTRATE: A carboxy-terminal amidated 12mer peptide with the sequence
SEQ ID NO: 5 TATKSGSTTKNR,
   derived from the amino acid sequence around serine 209 of the eukaryotic translation initiation factor 4E (eIF4E) has been synthesized and purified by high performance liquid chromatography (HPLC) to >95% (Thermo, Germany). The serine residue phosphorylated by Mnk kinases is underlined.
LIGAND: The peptide TATKSG-pS-TTKNR, containing an amidated carboxy-terminus and conjugated at the amino-terminus with the oxazine derived fluorophore depicted below was synthesized and used as ligand. 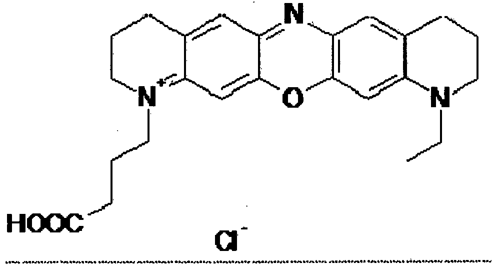
ANTIBODY: SPF New Zealand White Rabbits have been immunized according to standard protocols with the peptide NH2-CTATKSG-pS-TTKNR-CONH2, coupled to keyhole limpet hemocyanin (KLH). The immune globulin G (IgG) fraction was purified from serum of boosted animals by techniques known in the art. In brief, serum was subjected to protein A affinity chromatography. Eluted material was precipitated at 50% cold saturated ammonium sulfate, pellets dissolved and desalted. The resulting material was appropriate for use in below described assay without further antigen-specific purification.
ASSAY SETUP: Inhibition of kinase activity of Mnk1 and Mnk2a was assessed with the same assay system, using pre-activated GST-Mnk1 or GST-Mnk2a, respectively. The kinase reaction contains 30 µM substrate peptide, 20 µM ATP, 60 nM ligand and one of either 25 nM pre-activated Mnk1 or 2.5 nM pre-activated Mnk2a. The reaction buffer conditions are 16 mM HEPES/KOH pH 7.4, 8 mM MgCl₂, 0.4 mM DTT, 0.08 % (w/v) bovine serum albumin (BSA, Sigma, Germany, cat. no. A3059), 0.008% (w/v) Pluronic F127 (Sigma, Germany, cat. no. P2443), 3% (v/v) DMSO (Applichem, Germany, cat. no. A3006). The kinase reaction is at 30°C for 40 min. The kinase reaction is terminated by addition of 0.67 reaction volumes of 1 µM antibody in 20 mM HEPES/KOH pH 7.4, 50 mM ethylenediaminetetraacetic acid, disodium salt (EDTA, Sigma, Germany, cat. no. E5134), 0.5 mM DTT, 0.05% (w/v) polyoxyethylene-sorbitan monolaureate (Tween 20, Sigma, Germany, cat. no. P7949). After 1 h equilibration time at room temperature, samples are subjected to fluorescence polarization measurement. The fluorescence polarization readout was generated on an Analyst AD multimode reader (Molecular Devices, Sunnyvale, CA, USA) equipped with a DLRP650 dichroic mirror (Omega Opticals, Brattleboro, VT, USA, cat. no. XF2035), a 630AF50 band pass filter (Omega Opticals, Brattleboro, VT, USA, cat. no. XF1069) on the excitation and a 695AF55 band pass filter on the emission side (Omega Opticals, Brattleboro, VT, USA, cat. no. XF3076).

The activity of Mnk proteins can be assayed also by other in vitro kinase assay formats. For example, suitable kinase assays have been described in the literature in Knauf et al., Mol Cell Biol. 2001 Aug;21(16):5500-11 or in Scheper et al., Mol Cell Biol. 2001 Feb;21 (3):743-54. In general, Mnk kinase assays can be performed such that a Mnk substrate such as a protein or a peptide, which may or may not include modifications as further described below, or others are phosphorylated by Mnk proteins having enzymatic activity in vitro. The activity of a candidate agent can then be determined via its ability to decrease the enzymatic activity of the Mnk protein. The kinase activity may be detected by change of the chemical, physical or immunological properties of the substrate due to phosphorylation.

In one example, the kinase substrate may have features, designed or endogenous, to facilitate its binding or detection in order to generate a signal that is suitable for the analysis of the substrates phosphorylation status. These features may be, but are not limited to, a biotin molecule or derivative thereof, a glutathione-S-transferase moiety, a moiety of six or more consecutive histidine residues, an amino acid sequence or hapten to function as an epitope tag, a fluorochrome, an enzyme or enzyme fragment. The kinase substrate may be linked to these or other features with a molecular spacer arm to avoid steric hindrance.

In another example the kinase substrate may be labelled with a fluorophore. The binding of the reagent to the labelled substrate in solution may be followed by the technique of fluorescence polarization as it is described in the literature. In a variation of this example, a fluorescent tracer molecule may compete with the substrate for the analyte to detect kinase activity by a technique which is know to those skilled in the art as indirect fluorescence polarization.

In yet another example, radioactive gamma-ATP is used in the kinase reaction, and the effect of the test agent on the incorporation of radioactive phosphate in the test substrate is determined relative to control conditions.

It has been shown that the compounds of the invention exhibit low IC₅₀ values in *in vitro* biological screening assays as described in example 2a for inhibition of Mnk 1 and/or Mnk 2 kinase activity. The following table contains the test results for exemplary compounds.

### MNK2 Inhibition:

Example 8: 11 nM
Example 10: 8 nM
Example 13: 10 nM
Example15: 9 nM
Example 16.2: 16 nM
Example 18: 58 nM

(The structure of these Examples is given in the experimental section.)

### Examples

### Intermediate I

### 2-(6-Chloro-5-methyl-thieno[2,3-d]pyrimidin-4-ylamino)-5-fluorophenol

### I.1 5-Methyl-thieno[2,3-d]-pyrimidin-4-ol

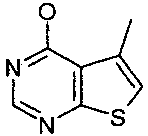

2-Amino-thiophene-3-carboxylic acid ethyl ester (25 g) was dissolved in formamide (200 ml), heated at 180°C for 6h and stirred at 150°C overnight. Then it was cooled to ambient temperature. The precipitate was filtered and washed with Et₂O then dissolved in hot ethylacetate and methanol. Charcoal was added, stirred for a few minutes, filtered and concentrated in vacuo. The residue was stirred with dichloromethane: methanol 50:1 and then washed with Et₂O, filtered and dried.
- Yield:: 10,40 g
- ESI mass spectrum:: m/z = 167 (M+H)⁺

### I.2 6-Chloro-5-methyl-thieno[2,3-d]pyrimidin-4(3H)-one

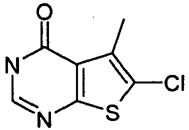

5-Methylthieno[2,3-d]-pyrimidin-4-ol (500 mg) and N-Chlorsuccinimide (480 mg) was dissolved in glacial acetic acid (20 ml) and heated at 95°C for 1,5h. Then the reaction mixture was concentrated in vacuo. The residue was stirred with water, filtered and washed again with water and methanol and dried.
- Yield:: 530 mg
- ESI mass spectrum:: m/z = 201 (M+H)⁺

### I.3 4,6-Dichloro-5-methyl-thieno[2,3-d]pyrimidine

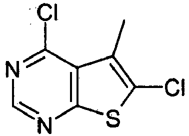

6-Chloro-5-methylthieno[2,3-d]pyrimidin-4(3H)-one (520 mg) was dissolved in 20 ml thionylchlorid, 0,2 ml DMF was added and the reaction mixture was heated under reflux for 2h. Then the mixture was evaporated, the residue was dissolved in dichloromethane and stirred with water for a few minutes. The organic layer was separated and concentrated in vacuo to give the crude product, which was used directly in the next step.
- Yield:: 560 mg

### I.4 2-(6-Chloro-5-methyl-thieno[2,3-d]pyrimidin-4-ylamino)-5-fluorophenol

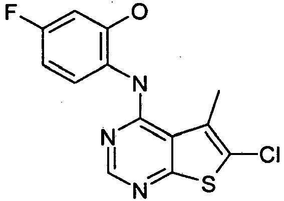

4,6-Dichloro-5-methyl-thieno[2,3-d]pyrimidine (560 mg) and 2-amino-5-fluorophenol (325 mg) were dissolved in 20 ml dioxane and 90 mg p-toluenesulfonic acid was added. The reaction mixture was heated at 140°C under microwave irradiation for 15 minutes, then it was cooled to ambient temperature and the resultant precipitate was filtered, washed with dioxane, methanol and Et₂O to yield the title compound.
- Yield:: 460 mg
- ESI mass spectrum:: m/z = 310 (M+H)⁺

### Intermediate II

### trans-3-(2-Amino-5-fluoro-ohenoxy)-cyclohexanol

### II.1 tert-Butyl-[trans-3-(5-fluoro-2-nitro-phenoxy)-cyclohexyloxy]-dimethylsilane

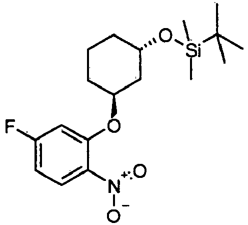

Diisopropyl-azodicarboxylate (30.2 ml) was added dropwise to triphenylphosphine (39.9 g) in THF (500 ml) and stirred at ambient temperature for 30 minutes. 5-Fluoro-2-nitrophenol was added portionwise and the mixture was stirred for further 30 minutes. After the addition of 3-(*tert*-butyl-dimethyl-silanyloxy)-cyclohexanol (30.0 g) it was stirred overnight. Further diisopropyl-azodicarboxylate and triphenylphosphine were added and after 2 hours the reaction mixture was poured into water and extracted with dichloromethane. The combined organic layers were washed with water, dried over Na₂SO₄ and concentrated in vacuo. The crude product was washed with petroleum ether and filtered. The filtrate was concentrated and purified by chromatography (silica gel, cyclohexane:dichloromethane 90/10-75/25) to separate the diastereomers and to yield the title compound as a racemic mixture.
- Yield:: 11.4 g
- ESI mass spectrum:: m/z = 370 (M+H)⁺

### II.2 2-[trans-3-(tert-Butyl-dimethyl-silanyloxy)-cyclohexyloxy]-4-fluoroaniline

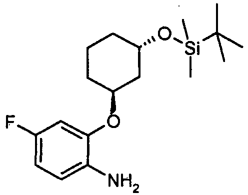

A mixture of *tert*-butyl-[trans-3-(5-fluoro-2-nitro-phenoxy)-cyclohexyloxy)-dimethylsilane (9.2 g) and Raney nickel (0.9 g) in MeOH (200 ml) was stirred for 20 hours under a hydrogen atmosphere (3.0 bar). The catalyst was removed by filtration and the mixture was concentrated in vacuo to yield the title compound as a racemic mixture.
- Yield:: 8.1 g
- ESI mass spectrum:: m/z = 340 (M+H)⁺

### II.3 trans-3-(2-Amino-5-fluoro-phenoxy)-cyclohexanol

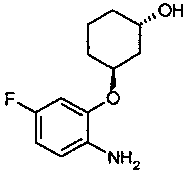

2-[trans-3-(*tert*-Butyl-dimethyl-silanyloxy)-cyclohexyloxy]-4-fluoroaniline (250 mg) was stirred in 1.25 M HCl in MeOH (10 ml) at ambient temperature for 30 minutes. The reaction mixture was concentrated in vacuo; the residue was basified with 1 M sodium hydroxide solution and extracted with ethyl acetate. The combined organic layers were dried over Na₂SO₄ and concentrated in vacuo to yield the title compound as a racemic mixture.
- Yield:: 140 mg
- ESI mass spectrum:: m/z = 226 (M+H)⁺

### Intermediate III

### 2-(1,3-Difluoropropan-2-yloxy)-4-fluoroaniline

### III. 1 2-(1,3-Difluoropropan-2-yloxy)-4-fluoro-1-nitrobenzene

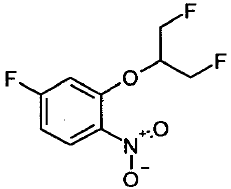

1,3-Difluoro-propan-2-ol (840 mg) was dissolved in 20 ml THF and cooled to 0°C. At this temperature LiHMDS (1M; 8.7 ml solution in THF) was added dropwise and the reaction mixture was stirred for 20 minutes. Then 2,4- difluoronitrobenzene (959 µl) in THF (5 ml) was added and stirring was continued overnight at ambient temperature. The reaction mixture was quenched with sat. aq. NH₄Cl solution and was extracted with dichloromethane ( DCM). The organic layer was concentrated in vacuo and the residue was purified by chromatography (silica gel, petroleum ether/EtOAc 1/1).
- Yield:: 1.30 g
- ESI mass spectrum:: m/z = 235 (M+H)⁺

### III. 2 2-(1,3-Difluoropropan-2-yloxy)-4-fluoroaniline

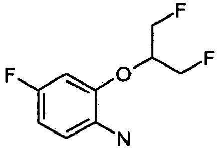

A mixture of 2-(1,3-difluoropropan-2-yloxy)-4-fluoro-1-nitrobenzene (5.0 g), methanol (500 ml) and Raney nickel (1.0 g) were stirred at ambient temperature for 6 hours under hydrogen atmosphere (5.0 bar). The catalyst was removed by filtration and the mixture was concentrated in vacuo to yield the title compound.
- Yield:: 4.42 g
- ESI mass spectrum:: m/z = 206 (M+H)⁺

### Intermediate IV

### (R)-3-(2-Amino-5-fluoro-phenoxy)-piperidine-1-carboxylic acid tert-butyl ester

### IV.1 (R)-3-(5-Fluoro-2-nitro-phenoxy)-piperidine-1-carboxylic acid tert-butyl ester

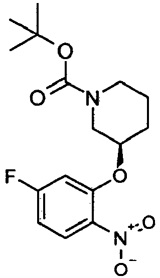

(R)-3-Hydroxy-piperidine-1-carboxylic acid *tert*-butyl ester (4.7 g) in THF (60 ml) was cooled to 0°C. At this temperature LiHMDS (1 M; 28.0 ml in THF) was added drop wise and the reaction mixture was stirred for 45 minutes. Then 2,4-difluoronitrobenzene (4.1 g) in THF (10 ml) was added and stirring was continued for 16 hours. The reaction mixture was quenched with sat. aq. NH₄Cl and concentrated in vacuo. The aqueous layer was adjusted to pH 3 with 10% aq. KHSO₄ and extracted with DCM. The organic layer was passed through a hydrophobic frit, concentrated and purified by chromatography (silica gel, hexan/EtOAc 4/1).
- Yield:: 6.7 g

### IV.2 (R)-3-(2-Amino-5-fluoro-phenoxy)-piperidine-1-carboxylic acid tert-butyl ester

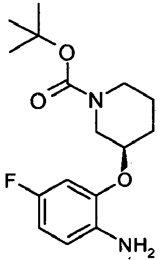

A mixture of (R)-3-(5-fluoro-2-nitro-phenoxy)-piperidine-1-carboxylic acid *tert*-butyl ester (6.7 g), ammonium formate (6.2 g) and palladium on carbon (1.0 g) in MeOH (80 ml) was stirred for 90 minutes at ambient temperature. The catalyst was removed by filtration and the mixture was concentrated in vacuo. The crude mixture was dissolved in DCM and washed with water. The organic layer was dried over Na₂SO₄ and concentrated in vacuo to yield the title compound.
- Yield:: 6.1 g

### Intermediate V

### trans-4-Fluoro-2-(4-Hydroxycyclohexyloxy)-aniline

### V.1 4-Fluoro-2-(4-hydroxycyclohexyloxy)-nitrobenzene

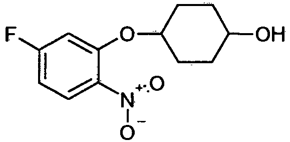

To a solution of 1,4-cyclohexanediol (100 g) in THF (1000 ml) was added NaH (60% in mineral oil; 38.5 g). The mixture was refluxed for 1 h. 2,4-Difluoronitrobenzene (48.0 ml) was added dropwise during 1 h and the mixture was refluxed for another 2 days, concentrated in vacuo, dissolved in water and extracted several times with DCM. The combined organic layers were washed with brine, dried with magnesium sulfate, filtered and concentrated in vacuo. The residue was purified by column chromatography (silica gel; DCM /MeOH 100:0 => 98:2) and yielded the mixture of isomers as a brownish oil.
- Yield:: 49.2 g
- ESI mass spectrum:: m/z = 273 (M+NH₄)⁺

### V.2 trans-4-Fluoro-2-(4-hydroxycyclohexyloxy)-nitrobenzene

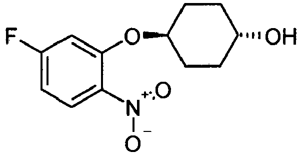

The cis/trans mixture from V.1 was separated into the isomers by SFC chromatography: Column: Daicel OJH 250 mm x 4.6 mm;
Mobile phase: CO₂/ Methanol 85:15 (with addition of 0.2% Diethyl amine)
Eluting first: cis isomer; eluting second: trans isomer

### V.3 trans-4-Fluoro-2-(4-hydroxycvclohexyloxy)-aniline

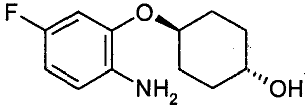

trans-4-Fluoro-2-(4-hydroxycyclohexyloxy)-nitrobenzene (2.0 g) in methanol (15 ml) was hydrogenated under 50 psi hydrogen using palladium on carbon (5%) as catalyst (300 mg) at ambient temperature for 2 h. The catalyst was filtered off; the solvent was removed in vacuo to yield the title compound.
- Yield:: 2.0 g
- ESI mass spectrum:: m/z = 226 (M+H)⁺

### Intermediate VI

### [2-(2-Amino-5-fluoro-phenoxy)-propyl]-carbamic acid tert-butyl ester

### VI.1 [2-(5-Fluoro-2-nitro-phenoxy)-propyl]-carbamic acid tert-butyl ester

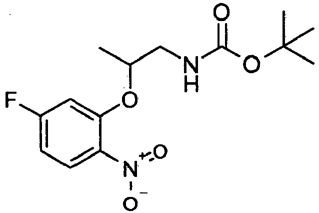

Triphenylphosphine (26.3 g) and di-tert-butyl-azodicarboxylate (23.2 g) were added under cooling to a mixture of 2-nitro-5-fluorophenol (10.5 g) and (2-hydroxy-propyl)-carbamic acid *tert*-butyl ester (14.8 g) in THF (70 ml) and stirred at ambient temperature for three days. The reaction mixture was concentrated in vacuo and the residue was purified by chromatography (silica gel, petroleum ether/DCM 1:1) to yield the desired compound.
- Yield:: 4.2 g
- ESI mass spectrum:: m/z = 315 (M+H)⁺

### VI.2 [2-(2-Amino-5-fluoro-phenoxy)-propyl]-carbamic acid tert-butyl ester

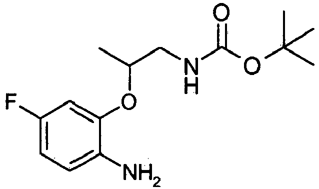

A mixture of [2-(5-fluoro-2-nitro-phenoxy)-propyl]-carbamic acid *tert*-butyl ester (3.1 g), MeOH (50 ml) and 10% palladium on carbon (300 mg) was stirred at ambient temperature for 2.5 h under hydrogen atmosphere (3 bar). The catalyst was removed by filtration and the filtrate was concentrated in vacuo.
- Yield:: 2.5 g
- ESI mass spectrum:: m/z = 285 (M+H)⁺

### Intermediate VII

### 4-Amino-3-ethoxybenzamide

### VII.1 3-Fluoro-4-nitrobenzamide

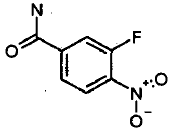

To a stirred solution of 3-fluoro-4-nitrobenzonitrile (15.0 g) and K₂CO₃ (25.0 g) in 100 ml acetone/ water (80:20) was added urea hydrogen peroxide (17.0 g) adduct. The reaction was stirred at room temperature. To the reaction mixture was added dichloromethane (100 ml) and water (500 ml) and the organic layer was separated. The aqueous layer was extracted with dichloromethane and the combined organic layers were washed with brine, filtered through celite and passed through a phase separator. The residue was concentrated in vacuo to yield an orange solid. The crude product was recrystallised from EtOAc/hexane to give the product.
- Yield:: 2.94 g

### VII.2 3-Ethoxy-4-nitrobenzamide

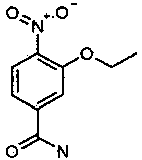

To ice cooled THF (8.0 ml) and Ethanol (990 µl) were added 0,64 g NaH. The mixture was stirred for 30 minutes, a solution of 3-fluoro-4-nitrobenzamide in THF (6.0 ml) was added to the suspension, the mixture was allowed to warm to ambient temperature and was stirred overnight. Then the reaction was quenched with water and the solvent was evaporated. The residue was dissolved in dichloromethane, washed with water and birne and evaporated in vacuo. The solid was recrystallized from EtOAc.
- Yield:: 1.25 g

### VII.3 4-Amino-3-ethoxybenzamide

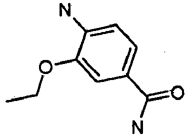

To a stirred solution of 3-ethoxy-4-nitrobenzamide in anhydrous MeOH (20.0 ml) was added ammonium formiate (2.22 g) followed by palladium on carbon (500 mg) and the reaction was heated at reflux for 2h. The reaction mixture was filtered through celite. The filtrate was evaporated to yield the title compound.
- Yield:: 1.52 g
- ESI mass spectrum:: m/z = 311 (M+H)⁺

### Intermediate VIII

### [3-(3-Amino-pyridin-2-yloxy)-butyl]-carbamic acid tert-butyl ester

### VIII.1 [3-(3-Nitro-pyridin-2-yloxy)-butyl]-carbamic acid tert-butyl ester

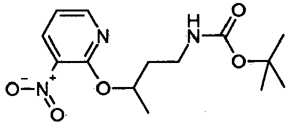

To a stirred mixture of KOH (7.07 g), potassium carbonate (4.36 g) and (3-hydroxy-butyl)-carbamic acid *tert*-butyl ester (8.52 g) in toluene (100 ml) was added 2-chloro-3-nitro-pyridine (5.00 g) followed by tris(3,6-dioxaheptyl)amine (1.03 g). The reaction mixture was stirred at ambient temperature for 2 h and concentrated in vacuo. The crude product was dissolved in a mixture of dichloromethane and water. The organic layer was separated, dried and concentrated. The residue was purified by chromatography (silica gel, DCM/MeOH 30/1) to yield the title compound.
- Yield:: 1.20 g
- ESI mass spectrum:: m/z = 312 (M+H)⁺

### VIII.2 [3-(3-Amino-pyridin-2-yloxy)-butyl]-carbamicacid tert-butyl ester

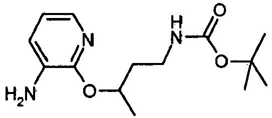

A mixture of [3-(3-nitro-pyridin-2-yloxy)-butyl]-carbamic acid *tert-*butyl ester (1.20 g), MeOH (30 ml) and Raney-Ni (150 mg) was stirred at ambient temperature overnight under hydrogen atmosphere (3 bar). The catalyst was removed by filtration and the filtrate was concentrated invacuo.
- Yield:: 1.1 g
- ESI mass spectrum:: m/z = 282 (M+H)⁺

### Intermediate IX

### cis-4-Methoxy-cyclohexanol

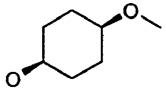

### IX.1. 4-Methoxycyclohexanol (mixture of cis and trans isomers)

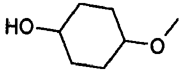

4-Methoxyphenol (100 g) in ethanol (759 ml) was hydrogenated under 50 psi hydrogen using Nishimura catalyst (10.0 g) at room temperature for 4.5 h. The catalyst was filtered off; the solvent was removed in vacuo to yield the desired product as 95% pure yellowish liquid.
- Yield:: 114 g
- ESI mass spectrum:: m/z = 131 (M+H)⁺

### IX.2. cis-4-Methoxycyclohexanol

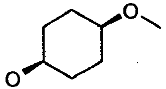

To a mixture of 5 g aluminiumtrichloride (37.5 mmol) and 9.32 ml of lithiumaluminiumhydride in diethylether (1 M) is added a solution of 4.8 g 4-methoxy-cyclohexanol (36.8 mmol ,cis/trans-mixture) in 5 ml of diethylether. The reaction mixture is stirred for 20 minutes at room temperature and then concentrated in vacuo. Diethylether is added and decanted off several times. Diethylether is added to the residue and then sulfuric acid (10%) is added until both phases are clear. The organic phase is separated and extracted consecutively with water and saturated sodium bicarbonate solution. The organic phase is dried.
- Yield:: 1.33 g (28%)
- ESI mass spectrum:: m/z = 131 (M+H)⁺

### Intermediate X

### 3-(6-Chloro-5-methyl-thieno[2,3-d]pyrimidin-4-ylamino)-pyridin-2-ol

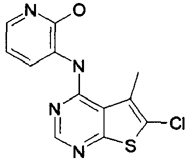

Prepared analogously to intermediate I.4 from 3-amino-pyridin-2-ol and intermediate I.3. The residue was purified by chromatography (silica gel, DCM/MeOH 30/1) to yield the title compound.
- Yield:: 0.35 g (52 %)

### Intermediate XI

### 4-(2-Amino-5-carbamoyl-phenoxy)-piperidine-1-carboxylic acid tert-butyl ester

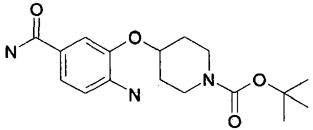

### XI.1. 4-(2-Nitro-5-carbamoyl-phenoxy)-piperidine-1-carboxylic acid tert-butyl ester

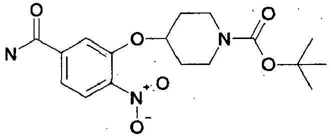

Prepared analogously to example VII.2 from 3-fluoro-4-nitrobenzamide and 4-hydroxy-piperidine-1-carboxylic acid *tert*-butyl ester.
- Yield:: 1.7 g (61%)
- ESI mass spectrum:: m/z = 366 (M+H)⁺

### XI.2. 4-(2-Amino-5-carbamoyl-phenoxy)-piperidine-1-carboxylic acid tert-butyl ester

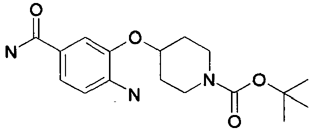

Prepared analogously to example VII.3 from 4-(2-nitro-5-carbamoyl-phenoxy)-piperidine-1-carboxylic acid *tert*-butyl ester.
- Yield:: 1.4 g (89%)

### Intermediate XII

### [4-trans-(3-Amino-pyridin-2-yloxy)-cyclohexyl]-carbamic acid tert-butyl ester

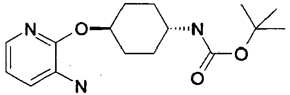

### XII.1. [4-trans-(3-Nitro-Pyridin-2-yloxy)-cyclohexyl]-carbamic acid tert-butyl ester

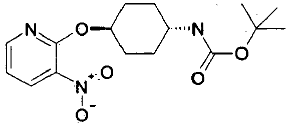

54 ml (54 mmol) LiHMDS (1 M in THF) were added at 0 °C to a solution of (4-hydroxy-cyclohexyl)-carbamic acid *tert*-butyl ester in 70 ml THF and stirred for 15 minutes. A solution of 7 g (49.2 mmol) 2-fluoro-3-nitro-pyrdine in a small amount of THF was added. The cooling was removed and the reaction mixture was stirred for 1.5 hours allowing to come to room temperature. Then the mixture was poured into water. Saturated ammoniumchloride solution was added. The mixture was extracted with ethylacetate. The organic phase was extracted with water and brine consecutively. Afterwards the organic phase was dried over sodiumsulfate.
- Yield:: 16.6 g (99.9%)
- ESI mass spectrum:: m/z = 338 (M+H)⁺

### XII.2 [4-trans-(3-Amino-pyridin-2-yloxy)-cyclohexyl]-carbamic acid tert-butyl ester

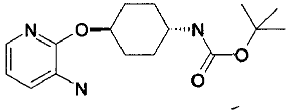

Prepared analogously to example II.2 from [4-trans-(3-nitro-pyridin-2-yloxy)-cyclohexyl]-carbamic acid *tert*-butyl ester.
- Yield:: 6.9 g (97%)
- ESI mass spectrum:: m/z = 308 (M+H)⁺

### Intermediate XIII

### 1-(2-Amino-5-fluoro-phenoxy)-2-methyl-propan-2-ol

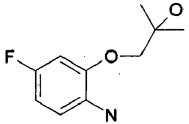

### XIII.1. 1-(2-Nitro-5-fluoro-phenoxy)-2-methyl-propan-2-ol

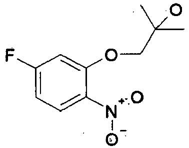

Prepared analogously to III.1 from 2,4-difluoronitrobenzene and 2-methyl-propane-1,2-diol using sodium hydride (60% in oil) instead of LiHMDS.
- Yield:: 1.9 g (93%)
- ESI mass spectrum:: m/z = 247 (M+NH4)⁺

### XIII.2 1-(2-Amino-5-fluoro-phenoxy)-2-methyl-propan-2-ol

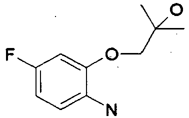

Prepared analogously to example VI.2 from 1-(2-nitro-5-fluoro-phenoxy)-2-methyl-propan-2-ol
- Yield:: 1.56 g (94%)
- ESI mass spectrum:: m/z = 200 (M+H)⁺

### Intermediate XV

### 3-(2-Amino-5-fluoro-phenoxy)-2-methyl-butan-2-ol.

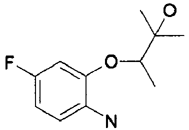

### XV.1. 3-(2-Nitro-5-fluoro-phenoxy)-2-methyl-butan-2-ol.

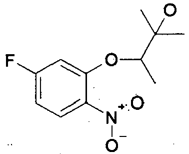

Prepared analogously to III.1 from 2,4-difluoronitrobenzene and 2-methyl-butane-2,3-diol using sodium hydride (60% in oil) instead of LiHMDS.
- Yield:: 1.7 g (85%)
- ESI mass spectrum:: m/z = 261 (M+NH4)⁺
- Rₜ (HPLC):: 1.18 min (method A_6)

### XV.2. 3-(2-Amino-5-fluoro-phenoxy)-2-methyl-butan-2-ol

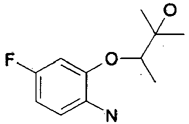

Prepared analogously to example VI.2 from 3-(2-nitro-5-fluoro-phenoxy)-2-methyl-butan-2-ol.
- Yield:: 1.45 g (97%)
- ESI mass spectrum:: m/z = 214 (M+H)⁺
- Rₜ (HPLC):: 0.68 min (method A_6)

### Example 1

### (trans-3-[2-(6-Chloro-5-methyl-thieno[2,3-d]pyrimidin-4-ylamino)-5-fluoro-phenoxy]-cyclohexanol

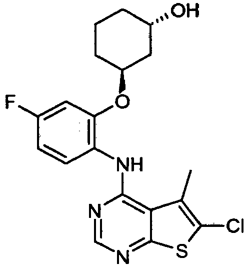

A mixture of 4,6-dichloro-5-methyl-thieno[2,3-d]pyrimidine (80 mg), trans-3-(2-amino-5-fluoro-phenoxy)-cyclohexanol (100 mg) and p-toluenesulfonic acid monohydrate (10 mg) in dioxane (2ml) was stirred under microwave irradiation at 140°C for 15 minutes. The reaction mixture was diluted with dichloromethane and 1 M aq. HCl. The organic layer was washed with 1 M sodium hydroxide solution and concentrated in vacuo. The residue was triturated with MeOH and filtered to yield the title compound.
- Yield:: 46 mg
- ESI mass spectrum:: m/z = 408 (M+H)⁺
- Rt (HPLC):: 2.19 min (method A_9)

### Example 2

### (6-Chloro-5-methyl-thieno[2,3-d]pyrimidin-4-yl)-[2-(trans-4-methylamino-cyclohexyloxy)-pyridin-3-yl]-amine

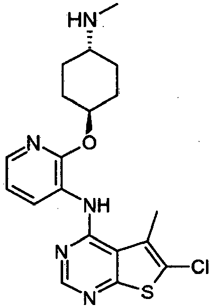

A mixture of 4,6-dichloro-5-methyl-thieno[2,3-d]pyrimidine (150mg), [4-(3-amino-pyridin-2-yloxy)-cyclohexyl]-methyl-carbamic acid *tert*-butyl ester (265 mg) and p-toluenesulfonic acid (20 mg) in dioxane (5 ml) was stirred at 100°C for 2 days. The reaction mixture was concentrated in vacuo and stirred with 25% TFA in DCM for 2 hours at ambient temperature. Then the reaction mixture was concentrated in vacuo again and purified by RP-chromatography ((H₂O+ 2% TFA)/MeOH: gradient 85/15 to 0/100). The fractions were collected, concentrated in vacuo and the residue was dissolved in DCM/MeOH and 1 M sodium hydroxide solution. The organic layer was dried and concentrated in vacuo to yield the title compound.
- Yield:: 120 mg
- ESI mass spectrum:: m/z = 404 (M+H)⁺
- Rₜ (HPLC):: 2.20 min (method A_4)

### Example 3

### N-{trans-4-[3-(6-Chloro-5-methyl-thieno[2,3-d]pyrimidine-4-ylamino)-pyridin-2-yloxy]-cyclohexyl}-N-methyl-methanesulfonamide

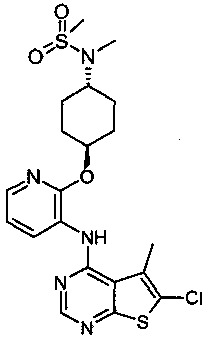

Triethylamine (40 µl) and methanesulfonyl chloride (12 µl) were added to (6-chloro-5-methyl-thieno[2,3-*d*]pyrimidine-4-yl)-[2-(trans-4-methylamino-cyclohexyloxy)-pyridin-3-yl]-amine (example 2; 50 mg) in DCM (1 ml) and stirred at ambient temperature for 2 hours. The reaction mixture was diluted with water and the dichloromethane was evaporated. The suspension was filtered and the resulting solid washed with MeOH and diethyl ether to yield the title compound.
- Yield:: 53 mg
- ESI mass spectrum:: m/z = 482 (M+H)⁺
- Rₜ (HPLC):: 3.65 min (method A_4)

### Example 4

### (6-Chloro-5-methyl-thieno[2,3-d]pyrimidin-4-yL)-[4-fluoro-2-(2-fluoro-1-fluoromethyl-ethoxy)-phenyl]-amine

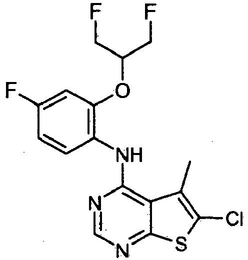

Prepared from 2-(1,3-difluoropropan-2-yloxy)-4-fluoroaniline (85 mg), 4,6-dichloro-5-methylthieno[2,3-d]pyrimidine (80 mg) and p-toluenesulfonic acid (10 mg) in dioxane (2ml) according to example 1.
- Yield:: 60 mg
- ESI mass spectrum:: m/z = 388 (M+H)⁺
- Rₜ (HPLC):: 2.27 min (method A_9)

### Example 5

### (6-Chloro-5-methyl-thieno[2,3-d]pyrimidin-4-yl)-[4-fluoro-2-((R)-piperidin-3-yloxy)-phenyl]-amine hydrochloride

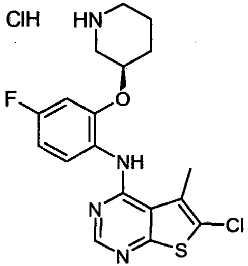

A mixture of (R)-3-(2-amino-5-fluoro-phenoxy)-piperidine-1-carboxylic acid *tert*-butyl ester (300 mg), 4,6-dichloro-5-methyl-thieno[2,3-d]pyrimidine (160 mg) and p-toluenesulfonic acid (20 mg) in dioxane (5ml) was stirred at 100°C for 3 days. The reaction mixture was concentrated in vacuo and the residue was stirred in 25% TFA in DCM for 2 hours at ambient temperature. The mixture was concentrated in vacuo again and purified by RP-chromatography ((H₂O+ 2% TFA)/MeOH: gradient 85/15 to 0/100). The fractions were collected, concentrated in vacuo and the residue was dissolved in methanol and methanolic HCl, concentrated in vacuo and triturated with acetone. The residue was filtered and washed with diethyl ether to yield the title product.
- Yield:: 125 mg
- ESI mass spectrum:: m/z = 393 (M+H)⁺
- Rₜ (HPLC):: 2.13 min (method A_4)

### Example 6

### 6-Chloro-5-methyl-thieno[2,3-d]pyrimidin-4-yl)-[4-fluoro-2-((R)-1-methanesulfonyl-piperidin-3-yloxy)-phenyl]-amine

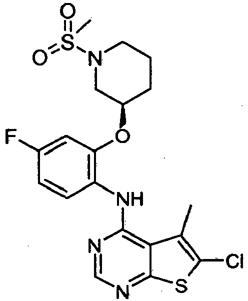

To a solution of (6-chloro-5-methyl-thieno[2,3-*d*]pyrimidin-4-yl)-[4-fluoro-2-((R)-piperidin-3-yloxy)-phenyl]-amine hydrochloride (example 5; 50 mg) in DCM (1 ml) was added triethylamine (40 µl) followed by methanesulfonyl chloride (11 µl). The reaction mixture was stirred at ambient temperature for 4 hours and quenched with DCM and water. The organic layer was dried and concentrated in vacuo to yield the title compound.
- Yield:: 25 mg
- ESI mass spectrum:: m/z = 471 (M+H)⁺
- Rₜ (HPLC):: 3.45 min (method A_4)

### Example 7

### 4-[2-(6-Chloro-5-methyl-thieno[2,3-d]pyrimidin-4-ylamino)-5-fluoro-phenoxy]-trans-cyclohexanol

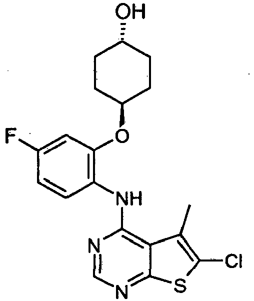

Prepared from trans-4-fluoro-2-(4-hydroxycyclohexyloxy)-aniline (100 mg), 4,6-dichloro-5-methylthieno[2,3-d]pyrimidine (80 mg) and p-toluenesulfonic acid monohydrate(10 mg) in dioxane (2ml) according to example 1.
- Yield:: 72 mg
- ESI mass spectrum:: m/z = 408 (M+H)⁺
- Rₜ (HPLC):: 3.47 min (method A_4)

### Example 8

### [2-(2-Amino-1-methyl-ethoxy)-4-fluoro-phenyl]-(6-bromo-5-methyl-thieno[2,3-d]pyrimidin-4-yl)-amine

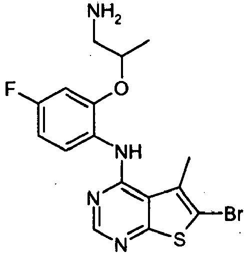

A mixture of 6-bromo-4-chloro-5-methyl-thieno[2,3-*d*]pyrimidine (500 mg) (Lit.: Robba et al. Bulletin de la Societe Chimique de France; 1975; 592 - 597), [2-(2-amino-5-fluoro-phenoxy)-propyl]-carbamic acid *tert*-butyl ester (590 mg) and DIPEA (1.0 ml) in dioxane (15 ml) was stirred at 100°C for three days. The reaction mixture was concentrated in vacuo, the residue was partitioned between DCM and water. Then the aq. layer was extracted with DCM/MeOH 9/1. The combined extracts were dried over Na₂SO₄ and concentrated in vacuo. The residue was stirred in 25% TFA in DCM for 1 hour at ambient temperature then concentrated again and purified by RP-chromatography ((H₂O+ 2% TFA)/MeOH: gradient 85/15 to 0/100). The fractions were collected, concentrated in vacuo and the residue was stirred in DCM and 1 M sodium hydroxide solution. The organic layer was dried and concentrated in vacuo. Finally the residue was triturated with diethylether and filtered to yield the title product.
- Yield:: 48 mg
- ESI mass spectrum:: m/z = 411 (M+H)⁺
- Rₜ (HPLC):: 2.17 min (method A_4)

### Example 9

### N-{2-[2-(6-Bromo-5-methyl-thieno[2,3-d]pyrimidin-4-ylamino)-5-fluoro-phenoxy]-propyl}-acetamide

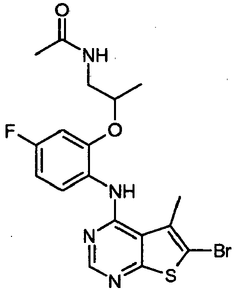

Acetyl chloride (6 µl) was added to a mixture of [2-(2-amino-1-methyl-ethoxy)-4-fluoro-phenyl]-(6-bromo-5-methyl-thieno[2,3-*d*]pyrimidin-4-yl)-amine (example 8, 20 mg) and triethylamine (8 µl) in DCM (0.5 ml) and stirred at ambient temperature overnight. The reaction mixture was diluted with water and the DCM was evaporated. The suspension was filtered; the resulting residue was washed with water, MeOH and diethyl ether to yield the title compound.
- Yield:: 18 mg
- ESI mass spectrum:: m/z = 453 (M+H)⁺
- Rₜ (HPLC):: 3.15 min (method A_4)

### Example 10

### N-{2-[2-(6-Bromo-5-methyl-thieno[2,3-d]pyrimidin-4-ylamino)-5-fluoro-phenoxy]-propyl}-methanesulfonamide

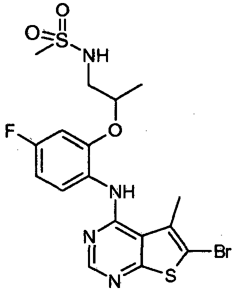

Prepared from [2-(2-amino-1-methyl-ethoxy)-4-fluoro-phenyl]-(6-bromo-5-methyl-thieno[2,3-*d*]pyrimidin-4-yl)-amine, (example 8; 20 mg), methanesulfonyl chloride (6 µl) and triethylamine (8 µl) in DCM (0.5 ml) according to example 9.
- Yield:: 19 mg
- ESI mass spectrum:: m/z = 489 (M+H)⁺
- Rₜ (HPLC):: 3.22 min (method A_4)

### Example 11

### (6-Bromo-5-methyl-thieno[2,3-d]pyrimidin-4-yl)-[-fluoro-2-(tetrahydro-pyran-4-yloxy)-phenyl]-amine

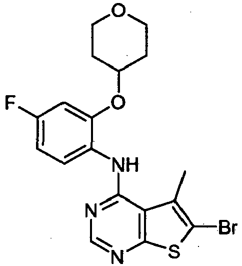

4-Fluoro-2-(tetrahydro-pyran-4-yloxy)aniline (225 mg) was added to a mixture of 6-bromo-4-chloro-5-methyl-thieno[2,3-*d*]pyrimidine (280 mg) and p-toluenesulfonic acid monohydrate (20 mg) in dioxane (5 ml) and stirred at reflux for 4 hours, at 80°C overnight and at 100°C for 2 hours. The reaction mixture was diluted with water, filtered and the residue was washed with water, dioxane and diethyl ether to give the title compound.
- Yield:: 340 mg
- ESI mass spectrum:: m/z = 438 (M+H)⁺
- Rt (HPLC):: 3.70 min (method A_4)

### Example 12

### {2-[2-(6-Chloro-5-methyl-thieno[2,3-d]pyrimidin-4-ylamino)-5-fluoro-phenoxy]-propyl}-carbamic acid tert-butyl ester

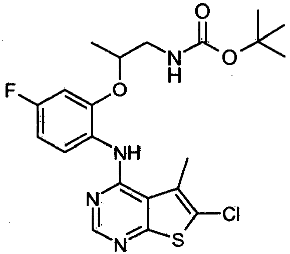

To a mixture of 4,6-dichloro-5-methyl-thieno[2,3-*d*]pyrimidine (460 mg), [2-(2-amino-5-fluoro-phenoxy)-propyl]-carbamic acid *tert*-butyl ester (390 mg) and triphenylphosphine (1.5 g) was added di-tert-butyl-azodicarboxylate (1.0 g) and stirred at room temperature for 4 hours. The reaction mixture was filtered through celite and washed with DCM and THF. The filtrate was concentrated in vacuo, the residue was triturated with MeOH, filtered and washed with MeOH and diethyl ether to give the title compound.
- Yield:: 470 mg
- ESI mass spectrum:: m/z = 467. (M+H)⁺
- Rₜ (HPLC):: 2.39 min (method A_9)

### Example 13

### [2-(2-Amino-1-methyl-ethoxy)-4-fluoro-phenyl]-(6-chloro-5-methyl-thieno[2,3-d]pyrimidin-4-yl)-amine trifluoroacetate

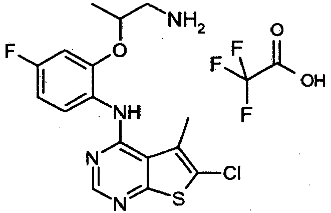

{2-[2-(6-Chloro-5-methyl-thieno[2,3-*d*]pyrimidin-4-ylamino)-5-fluoro-phenoxy]-propyl}-carbamic acid *tert*-butyl ester, (example 12, 350 mg) was stirred in 25% TFA in DCM at room temperature for 4 hours. The reaction mixture was concentrated to give the title compound.
- Yield:: 340 mg
- ESI mass spectrum:: m/z = 367 (M+H)⁺
- Rₜ (HPLC):: 1.79 min (method A_9)

### Example 14

### N-{2-[2-(6-Chloro-5-methyl-thieno[2,3-d]pyrimidin-4-ylamino)-5-fluoro-phenoxy]-propyl}-acetamide

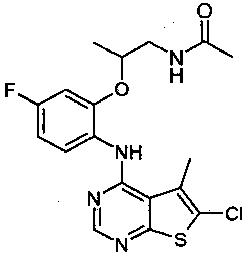

Acetyl chloride (16 µl) was added to a mixture of [2-(2-amino-1-methyl-ethoxy)-4-fluoro-phenyl]-(6-chloro-5-methyl-thieno[2,3-*d*]pyrimidin-4-yl)-amine trifluoroacetate (80 mg) and triethylamine (58 µl) in DCM (1.5 ml) and stirred at ambient temperature overnight. The reaction mixture was diluted with DCM and water. The organic layer was dried, concentrated in vacuo and the residue was triturated with diethyl ether, filtered and washed with MeOH and diethyl ether to yield the title compound.
- Yield:: 48 mg
- ESI mass spectrum:: m/z = 409 (M+H)⁺
- Rt (HPLC):: 1.93 min (method A_9)

### Example 15

### N-{2-[2-(6-Chloro-5-methyl-thieno[2,3-d]pyrimidin-4-ylamino)-5-fluoro-phenoxy]-propyl}-methanesulfonamide

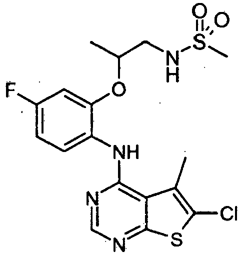

Prepared from [2-(2-Amino-1-methyl-ethoxy)-4-fluoro-phenyl]-(6-chloro-5-methyl-thieno[2,3-*d*]pyrimidin-4-yl)-amine trifluoroacetate, (example 13; 80 mg), methanesulfonyl chloride (17 µl) and triethylamine (58 µl) in DCM (1.5 ml) according to example 14.
- Yield:: 64 mg
- ESI mass spectrum:: m/z = 445 (M+H)⁺
- Rₜ (HPLC):: 2.00 min (method A_9)

### Example 16

### 4-(6-Chloro-5-methyl-thieno[2,3-d]pyrimidin-4-ylamino)-3-ethoxy-benzamide

### 16.1 3-Ethoxy-4-(5-methyl-thieno[2,3-d]pyrimidin-4-ylamino)-benzamide

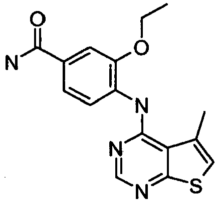

A mixture of 4-chloro-5-methyl-thieno[2,3-*d*]pyrimidine (1.2 g), 4-amino-3-ethoxy-benzamide (1.1 g) and p-toluenesulfonic acid monohydrate in i-PrOH (20 ml) was stirred at 100°C for 20 hours. The mixture was concentrated and the residue was heated in dioxane to reflux. The reaction mixture was quenched with ammonia and water, filtered and the residue was washed with water, diethyl ether and cyclohexane to yield the title compound.
- Yield:: 1.4 g

### 16.2 4-(6-Chloro-5-methyl-thieno[2,3-d]pyrimidin-4-ylamino)-3-ethoxy-benzamide

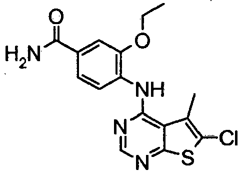

A mixture of 3-ethoxy-4-(5-methyl-thieno[2,3-d]pyrimidin-4-ylamino)-benzamide (100 mg) and N-chlorosuccinimide (45 mg) in acetic acid (1.5 ml) were stirred at 25°C for 1 hour and at 80°C for another hour. Then the reaction mixture was cooled, diluted with water, filtered and the residue was washed with diethyl ether. The -product was recrystallised from MeOH to yield the title compound.
- Yield:: 57 mg
- ESI mass spectrum:: m/z = 363 (M+H)⁺
¹H NMR (400MHZ; d₆DMSO; 20°C): δ 9.55 (s, 1H), 8.46 (s, 1H), 8.13 (d, 1H), 7.70 (s, 1H), 7.60 (d, 2H), 7.42 (d, 1H), 4.08 (m, 2H), 2.54 (s, 3H), 1.10 (m, 3H).

### Example 17

### 4-(6-Bromo-5-methyl-thieno[2,3-d]pyrimidin-4-ylamino)-3-ethoxy-benzamide

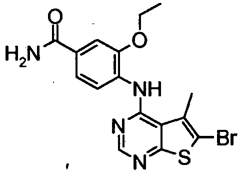

N-Bromosuccinimide (178 mg) followed by 2,2'-azobis(isobutyronitrile) (8 mg) were added to 3-ethoxy-4-(5-methyl-thieno[2,3-d]pyrimidin-4-ylamino)-benzamide, (example 16.1 328 mg) in dry carbon tetrachloride (5 ml) at reflux and stirred for 3 hours. The reaction mixture was filtered, the residue was washed with MeOH and purified by chromatography.
- Yield:: 15 mg
- ESI mass spectrum:: m/z = 408 (M+H)⁺
¹H NMR (400MHZ; d₆DMSO; 20°C): δ 8.80 (d, 1 H), 8.65 (s, 2H), 7.97 (s, 1 H), 7.61 (d, 2H), 7.33 (s, H), 4.25 (m, 2H), 2.79 (s, 3H), 1.50 (m, 3H).

### Example 18

### (6-Bromo-5-methyl-thieno[2,3-d]pyrimidin-4-yl)-(4-fluoro-2-isopropoxy-phenyl)-amine

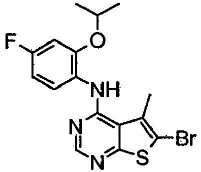

A mixture of 6-bromo-4-chloro-5-methyl-thieno[2,3-d]pyrimidine (456 mg), 4-fluoro-2-isopropoxy-phenylamine (293 mg) and p-toluenesulfonic acid (33 mg) in dioxane (6 ml) was stirred at 80°C for three days. The reaction mixture was quenched with EtOAc and 10% aq. K₂CO₃ and filtered. The residue was washed with further EtOAc and dried to yield the title compound.
- Yield:: 288 mg
- ESI mass spectrum:: m/z = 397 (M+H)⁺
¹H NMR (400MHZ; d₆DMSO; 20°C): δ 8.47 (s, 2H), 8.34 (s, 1 H), 7.17-7.03 (m, 1 H), 6.90-6.78 (m, 1 H), 4.88-4.76 (m, 1 H), 2.74 (s, 3H), 1.35 (d, 6H).

### Example 19

### [2-(3-Amino-1-methyl-propoxy)-pyridin-3-yl]-(6-chloro-5-methyl-thieno[2,3-d]pyrimidin-4-yl)-amine trifluoroacetate

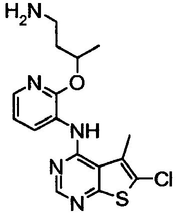

A mixture of [3-(3-Amino-pyridin-2-yloxy)-butyl]-carbamic acid *tert*-butyl ester (180 mg), 4,6-dichloro-5-methyl-thieno[2,3-*d*]pyrimidine (140 mg) and p-totuenesuifonic acid (12 mg) in dioxane (5 ml) was stirred at 100°C for 24 hours. The reaction mixture was concentrated in vacuo and the residue was purified by chromatography (silica gel, DCM/MeOH 30/1) to give the title compound.
- Yield:: 8 mg
- ESI mass spectrum:: m/z = 364 (M+H)⁺
- Rₜ (HPLC):: 2.44 min (method A_10)

### Example 20

### N-{3-[3-(6-Chloro-5-methyl-thieno[2,3-d]pyrimidin-4-ylamino)-pyridin-2-yloxy]-butyl}-methanesulfonamide

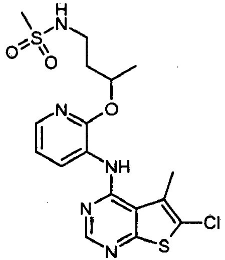

Methanesulfonyl chloride (15 µl) was added to a mixture of [2-(3-Amino-1-methyl-propoxy)-pyridin-3-yl]-(6-chloro-5-methyl-thieno[2,3-*d*]pyrimidin-4-yl)-amine trifluoroacetate (90 mg) and DIPEA (70 µl) in DCM and stirred at ambient temperature 30 min. The reaction mixture was concentrated and purified by chromatography (silica gel, DCM/MeOH 30/1) to give the title compound.
- Yield:: 23 mg
- ESI mass spectrum:: m/z = 442 (M+H)⁺
- Rₜ (HPLC):: 2.00 min (method A_10)

### Example 21

### (6-Chloro-5-methyl-thieno[2,3-d]pyrimidin-4-yl)-[2-(trans-4-methoxy-cyclohexyloxy)-pyridin-3-yl]-amine

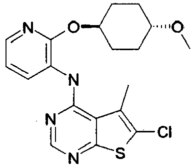

To a mixture of 80 mg (0.27 mmol) 3-(6-chloro-5-methyl-thieno[2,3-*d*]pyrimidin-4-ylamino)-pyridin-2-ol, 50 mg (0.38 mmol) cis-4-methoxy-cyclohexanol and 270 mg triphenylphosphine (polymer bound, 0.81 mmol) in THF was added 180 mg (0.78 mmol) diisobutylazodicarboxylate. The mixture was stirred for 18 hours at room temperature. The mixture was then filtered over Celite and washed with methanol, DMF and dichloromethane. The organic phases were concentrated in vacuo. Purification was achieved by HPLC (method P_1).
- Yield:: 18 mg (16%)
- ESI mass spectrum:: m/z = 405 (M+H)⁺
- Rₜ (HPLC):: 2.51 min (method A_9)

### Example 22

### (6-Chloro-5-methyl-thieno[2,3-d]pyrimidin-4-yl)-[2-(trans-4-hydroxy-cyclohexylooxy)-pyridin-3-yl]-amine

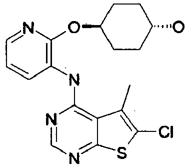

Prepared analogously to example 21 from 3-(6-chloro-5-methyl-thieno[2,3-*d*]pyrimidin-4-ylamino)-pyridin-2-ol and cis-4-hydroxy-cyclohexanol.
- ESI mass spectrum:: m/z = 391 (M+H)⁺
- Rₜ (HPLC):: 2.02 min (method A_9)

### Example 23 (Reference Example)

### 4-(6-Chloro-5-methyl-thieno[2,3-d]pyrimidin-4-ylamino)-3-(tetrahydro-pyran-4-yloxy)-benzamide,

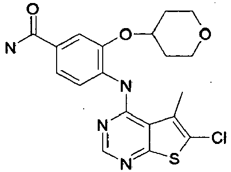

Prepared analogously to example I.4 from 4,6-dichloro-5-methyl-thieno[2,3-d]pyrimidine and 4-amino-3-(tetrahydro-pyran-4-yloxy)-benzamide.
- ESI mass spectrum:: m/z = 419 (M+H)⁺
- Rₜ (HPLC):: 1.73 min (method A_9)

### Example 24

### 4-(6-Chloro-5-methyl-thieno[2,3-d]pyrimidin-4-ylamino)-3-isopropoxy-benzamide

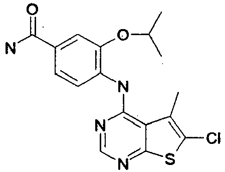

Prepared analogously to example 1.4 from 4,6-dichloro-5-methyl-thieno[2,3-d]pyrimidine and 4-amino-3-isopropoxy-benzamide.
- Yield:: 52 mg (38%)
- ESI mass spectrum:: m/z = 377 (M+H)⁺
- Rₜ (HPLC):: 1.91 min (method A_9)

### Example 25

### 4-(6-Chloro-5-methyl-thieno[2,3-d]pyrimidin-4-ylamino)-3-(piperidin-4-yloxy)-benzamide,

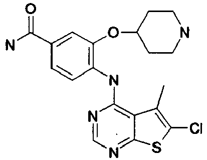

Prepared analogously to example 1.4 from 4,6-dichloro-5-methyl-thieno[2,3-d]pyrimidine and intermediate XI.
- Yield:: 35 mg (18%)
- ESI mass spectrum:: m/z = 418 (M+H)⁺

### Example 26

### (6-Chloro-5-methyl-thieno[2,3-d]pyrimidin-4-yl)-[4-fluoro-2-(4-methoxy-cyclohexyloxy)-phenyl]-amine

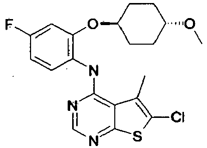

Prepared analogously to example 21 from intermediate I.4 and intermediate IX.
- Yield:: 4 mg (6%)
- ESI mass spectrum:: m/z = 422 (M+H)⁺
- Rₜ (HPLC):: 4.03 min (method A_4)

### Example 27

### [2-(4-Amino-cyclohexyloxy)-pyridin-3-yl]-(6-chloro-5-methyl-thieno[2,3-d]pyrimidin-4-yl)-amine

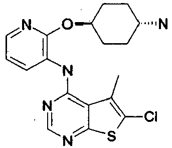

A mixture of 100 mg (0.45 mmol) 4,6-dichloro-5-methyl-thieno[2,3-d]pyrimidine, 170 mg (0.55 mmo) intermediate XII and 15 mg p-toluenesulfonic acid in 5 ml dioxane were stirred for 15 minutes at 130 °C in the microwave oven. Then the mixture was concentrated in vacuo. 10 ml of a mixture of methylenchloride and 20 % trifluoroacetic acid were added to the residue and stirred for 1.5 hours at room temperature. The mixture was concentrated. The residue was purified by preparative HPLC (method P_2).
- Yield:: 46 mg (20%)
- ESI mass spectrum:: m/z = 390 (M+H)⁺
- Rₜ (HPLC):: 2.27 min (method A_5)

### Example 28

### 1-[2-(6-Bromo-5-methyl-thieno[2,3-d]pyrimidin-4-ylamino)-5-fluoro-phenoxy]-2-methyl-propan-2-ol,

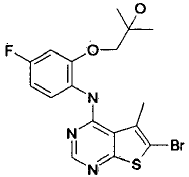

Prepared analogously to I.4 from 6-bromo-4-chloro-5-methyl-thieno[2,3-*d*]pyrimidine and 1-(2-amino-5-fluoro-phenoxy)-2-methyl-propan-2-ol
- Yield:: 430 mg (53%)
- ESI mass spectrum:: m/z = 427 (M+H)⁺
- Rₜ (HPLC):: 1.59 min (method A_6)

### Example 29

### 3-[2-(6-Bromo-5-methyl-thieno[2,3-d]pyrimidin-4-ylamino)-5-fluoro-phenoxy]-2-methyl-butan-2-ol

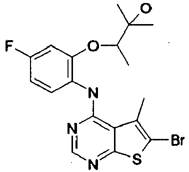

Prepared analogously to I.4 from 6-bromo-4-chloro-5-methyl-thieno[2,3-*d*]pyrimidine and 3-(2-amino-5-fluoro-phenoxy)-2-methyl-butan-2-ol.
- Yield:: 460 mg (55%)
- ESI mass spectrum:: m/z = 440 (M+H)⁺
- Rₜ (HPLC):: 1.64 min (method A_6)

The HPLC data provided in the examples described below were obtained as follows:

### Method A_9:

Waters ZQ 2000; Waters 1515 Pumpe; Waters PDA 996 Detektor; Waters 2747 Injektor
DAD 200-420 nm
mobile phases:
A: water with 0.10% formic acid
B: acetonitrile with 0.10% formic acid

| time in min | %A | %B | flow rate in ml/min |
|---|---|---|---|
| 0.00 | 95 | 5 | 1.50 |
| 2.00 | 0 | 100 | 1.50 |
| 2.50 | 0 | 100 | 1.50 |
| 2.60 | 95 | 5 | 1.50 |

### Stationary phase:

X-terra MS C18; 4.6x30mm*2.5µm

### Method A_4

Waters ZQ 2000; Waters 1515 Pump; Waters PDA 996 Detektor; Waters 2747 Injektor
DAD 200-420 nm
mobile phases:
A: water with 0.10% formic acid
B: acetonitrile with 0.10% formic acid

| time in min | %A | %B | flow rate in ml/min |
|---|---|---|---|
| 0.00 | 95 | 5 | 1.5 |
| 0.10 | 95 | 5 | 1.5 |
| 3.10 | 2 | 98 | 1.5 |
| 4.50 | 2 | 98 | 1.5 |
| 5.00 | 95 | 5 | 1.5 |

### Stationary phase:

X-terra MS C18; 4.6x30mm*2.5µm

### Method A_5

Waters ZQ2000; Waters 1515 Pump, Waters PDA 996 Detektor, Waters 2747 Injektor

| | | | |
|---|---|---|---|
| mobile phase: | A water+ 0,1% formic acid | | |
| | B acetonitrile + 0,1% formic acid | | |
| Gradient: | | | |
| time in min | %A | %B | flowrate in mL/min |
| time in min | %A | %B | flow rate in mL/min |
| 0.00 | 95.0 | 5.0 | 1.5 |
| 2.00 | 0.0 | 100 | 1.5 |
| 2.50 | 0.0 | 100 | 1.5 |
| 2.60 | 95.0 | 5.0 | 1.5 |

stationary phase: X-terra^{™} MS C18 2,5 µm 4,6 mm x 30 mm
column temperature ca. 25°C

### Method A_6

Agilent 1200, MS G6140A, binäre Pumpe, DAD 190-400 nm
Fragmentor 70, Gain EMV 1.0 Mass Range 100 - 1000

| **Column type** | **Column size** | **Flow rate** [ml/min] | **Gradient** |
|---|---|---|---|
| Agilent StableBond SB-C18 | 4.6x30mm 1.8µm | 3.00 | 0.00: 10%B; 1.80: 100%B; 2.00: 100%B; 2.15: 10%B; 2.35: 10%B |

- Solvent A :: Wasser + 0.1%TFA
- Solvent B :: Methanol
- Temperature:: 60°C

### Method P_1

Preparative column: xterra column (Waters technologies), MSC18 xterra ODB 5 µm 30x100 mm, column temperature 25 °C
mobile phases:
A: water / trifluoroacetic acid 99.8/0.2
B: methanol

| time in min | %A | %B | flow rate in ml/min |
|---|---|---|---|
| 0.00 | 46.0 | 54.0 | 55.00 |
| 2.00 | 46.0 | 54.0 | 55.00 |
| 2.50 | 36.0 | 64.0 | 55.00 |
| 9.50 | 0 | 100 | 55.00 |
| 10.00 | 0 | 100 | 55.00 |
| 12.00 | 0 | 100 | 55.00 |
| 12.50 | 0 | 100 | 0 |

### Method P_2

preparative column: xterra^{™} columns (Waters technologies) MSC18 xterra ODB™ 5µm 30x100mm column temperature 25°C

**Gradient:**

| time in min | %A | %B | flow rate in ml/min |
|---|---|---|---|
| 0.00 | 90.0 | 10.0 | 63.00 |
| 2.00 | 90.0 | 10.0 | 63.00 |
| 2.50 | 67.0 | 33.0 | 63.00 |
| 9.50 | 33.0 | 67.0 | 63.00 |
| 10.00 | 5.00 | 95.0 | 63.00 |
| 12.00 | 5.00 | 95.0 | 63.00 |
| 12.50 | 90.0 | 10.0 | 63.00 |
| 14.50 | 90.0 | 10.0 | 63.00 |
| 15.00 | 90.0 | 10.0 | 0 |

## Claims

1. A compound of general formula 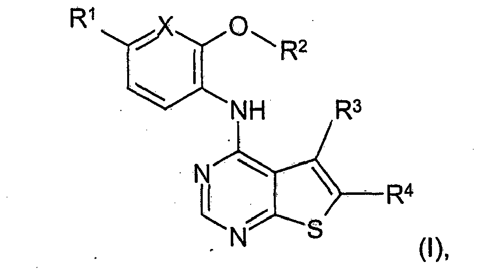 wherein
X is CH or N,
R¹ is a hydrogen or halogen atom, CN or CONH₂,
R² is a straight chain or branched C₁₋₆ alkyl group that is optionally substituted from position 2 onwards by one or two OH, C₁₋₄ alkoxy or NH₂, or that is substituted in any position by one to three F, or by a CO₂H, CONH₂, CONH(C₁₋₃ alkyl), CON(C₁₋₃ alkyl)₂, CONRR', SO₂NH₂, SO₂NH(C₁₋₃ alkyl) or SO₂N(C₁₋₃ alkyl)₂ group,
wherein the rests R and R' together with the N atom to which they are attached form a 3-8 membered ring, wherein one CH₂ group may be replaced by O, S, SO, SO₂ or N, and wherein any carbon atom other than one attached to the nitrogen atom, may be substituted by OH, F C₁₋₃ alkyl or NH₂;
a C₃₋₈ cycloalkyl group that is optionally substituted by one or two OH, C₁₋₄ alkoxy or NH₂ on any carbon atom other than one attached to the oxygen atom; or by F, CO₂H, CONH₂, CONH(C₁₋₃ alkyl), CON(C₁₋₃ alkyl)₂, SO₂NH₂, SO₂NH(C₁₋₃ alkyl), SO₂N(C₁₋₃ alkyl)₂, SO₂(C₁₋₃ alkyl), (CH₂)ₘOR⁵; (CH₂)ₘN(R⁵)₂,
wherein the NH₂ groups mentioned above as substituent for the alkyl and cycloalkyl groups may optionally be independently substituted by C₁₋₃ alkyl, -(CH₂)ₘ-OH, -(CH₂)ₘ-OCH₃, -(CH₂)ₘ-CN, -(CH₂)ₘ-F, SO₂(C₁₋₃ alkyl), CO₂(C₁₋₄ alkyl) or CO(C₁₋₃ alkyl);
or a heterocyclyl system selected from any one of the following formulae: 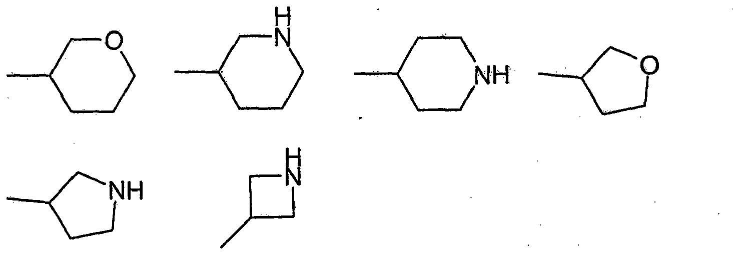 optionally substituted by one or more of R⁶,
wherein the hydrogen atom of the NH group may optionally be replaced by C₁₋₃ alkyl, SO₂(C₁₋₃ alkyl), CO₂(C₁₋₄ alkyl) or CO(C₁₋₃ alkyl);
R³ is a C₁₋₂ alkyl group;
R⁴ is F, Cl, Br, I or CN; .
R⁵ is selected from H and C₁₋₄ alkyl;
R⁶ is selected from OH, OR⁵ and N(R⁵)₂ on any carbon atom other than one attached to O or N; F; CO₂H; CON(R⁵)₂; SO₂N(R⁵)₂; SO₂R⁵; (CH₂)ₘOR⁵; (CH₂)ₘN(R⁵)₂; and
m is 1, 2 or 3;
or a salt thereof.

2. A compound of formula I according to claim 1, wherein
X is CH or N;
R¹ is a hydrogen or fluorine atom or a CONH₂ group,
R² is a straight chain or branched C₁₋₄ alkyl group that is optionally substituted from position 2 onwards by NH₂; or a straight chain or branched C₁₋₄ alkyl group that is substituted in any position by one to three F; or a straight chain or branched C₁₋₄ alkyl group that is substituted in any position by CONH₂, CONH(C₁₋₃ alkyl), CON(C₁₋₃ alkyl)₂ or CONRR',
wherein the rests R and R' together with the N atom to which they are attached form a 3-8 membered ring, wherein one CH₂ group may be replaced by O or N, and wherein any carbon atom may be substituted by OH, F or NH₂;
a C₅₋₇ cycloalkyl group that is optionally substituted by one or two OH, C₁₋₄ alkoxy or NH₂ on any carbon atom other than one attached to the oxygen atom,
wherein the NH₂ groups mentioned above as substituent for the alkyl and cycloalkyl groups may optionally be independently substituted by C₁₋₃ alkyl, -(CH₂)ₘ-OH, -(CH₂)ₘ-OCH₃, -(CH₂)ₘ-CN, -(CH₂)m₋F, SO₂(C₁₋₃ alkyl), CO₂(C₁₋₄ alkyl) or CO(C₁₋₃ alkyl);
or heterocyclyl systems selected from any one of the following formulae: 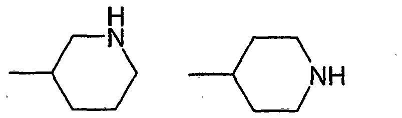 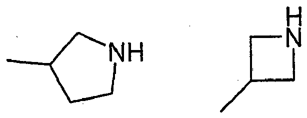 wherein the hydrogen atom of the NH group may optionally be replaced by SO₂(C₁₋₃ alkyl) or CO₂(C₁₋₄ alkyl);
R³ is a C₁₋₂ alkyl group;
R⁴ is F, Cl, Br or CN; and
m is 1, 2 or 3;
or a salt thereof.

3. A compound of formula I according to claim 2, wherein
XisCHorN,
R¹ is a hydrogen or fluorine atom or a CONH₂ group,
R² is a straight chain or branched C₁₋₄ alkyl group that is optionally substituted from position 2 onwards by NH₂, or that is substituted in any position by one to three F;
a cyclohexyl group that is optionally substituted by a OH, C₁₋₃ alkoxy or NH₂ on any carbon atom other than one attached to the oxygen atom,
wherein the NH₂ groups mentioned above as substituent for the alkyl and cycloalkyl groups may optionally be independently substituted by C₁₋₃ alkyl, SO₂(C₁₋₃ alkyl), CO₂(C₁₋₄ alkyl) or CO(C₁₋₃ alkyl);
or heterocyclyl systems selected from any one of the following formulae: 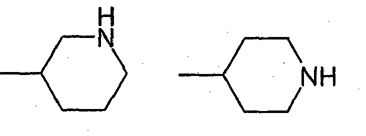 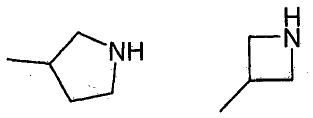 wherein the hydrogen atom of the NH group may optionally be replaced by SO₂(C₁₋₃ alkyl) or CO₂(C₁₋₄ alkyl);
R³ is a C₁₋₂ alkyl group and
R⁴ is F, Cl or Br,
or a salt thereof.

4. A compound of formula I according to any one of claims 1 to 3, wherein
X and R¹, R² and R⁴ are as defined in the previous claims and
R³ is CH₃,
or a salt thereof.

5. A compound of formula I according to any one of claims 1 to 3, wherein
R² to R⁴ are as defined in the previous claims,
X is CH and
R¹ is F or CONH₂,
or a salt thereof.

6. A compound of formula I according to any one of claims 1 to 3; wherein
R² to R⁴ are as defined in the previous claims,
X is N and
R¹ is H,
or a salt thereof.

7. A compound of formula I according to any one of claims 1 to 3, wherein
X and R¹ to R³ are as defined in the previous claims and
R⁴ is F, Cl or Br,
or a salt thereof.

8. A compound of formula I according to claim 7, wherein
X and R¹ to R³ are as defined in the previous claims and
R⁴ is Cl or Br,
or a salt thereof.

9. A Compound selected from:
a) (trans-3-[2-(6-Chloro-5-methyl-thieno[2,3-*d*]pyrimidin-4-ylamino)-5-fluoro-phenoxy]-cyclohexanol,
b) (6-Chloro-5-methyl-thieno[2,3-*d*]pyrimidin-4-yl)-[2-(trans-4-methylamino-cyclohexyloxy)-pyridin-3-yl]-amine,
c) (6-Chloro-5-methyl-thieno[2,3-*d*]pyrimidin-4-yl)-[4-fluoro-2-(2-fluoro-1-fluoromethyl-ethoxy)-phenyl]-amine,
d) (6-Chloro-5-methyl-thieno[2,3-*d*]pyrimidin-4-yl)-[4-fluoro-2-((R)-piperidin-3-yloxy)-phenyl]-amine,
e) 6-Chloro-5-methyl-thieno[2,3-*d*]pyrimidin-4-yl)-[4-fluoro-2-((R)-1-methanesulfonyl-piperidin-3-yloxy)-phenyl]-amine,
f) [2-(2-Amino-1-methyl-ethoxy)-4-fluoro-phenyl]-(6-bromo-5-methyl-thieno[2,3-*d*]pyrimidin-4-yl)-amine,
g) *N*-{2-[2-(6-Bromo-5-methyl-thieno[2,3-*d*]pyrimidin-4-ylamino)-5-fluoro-phenoxy]-propyl}-methanesulfonamide,
h) (6-Bromo-5-methyl-thieno[2,3-*d*]pyrimidin-4-yl)-[4-fluoro-2-(tetrahydro-pyran-4-yloxy)-phenyl]-amine,
i) [2-(2-Amino-1-methyl-ethoxy)-4-fluoro-phenyl]-(6-chloro-5-methyl-thieno[2,3-*d*]pyrimidin-4-yl)-amine,
j) *N*-{2-[2-(6-Chloro-5-methyl-thieno[2,3-*d*]pyrimidin-4-ylamino)-5-fluoro-phenoxy]-propyl}-acetamide,
k) *N*-{2-[2-(6-Chloro-5-methyl-thieno[2,3-*d*]pyrimidin-4-ylamino)-5-fluoro-phenoxy]-propyl}-methanesulfonamide,
l) 4-(6-Chloro-5-methyl-thieno[2,3-*d*]pyrimidin-4-ylamino)-3-ethoxy-benzamide,
m) 4-(6-Bromo-5-methyl-thieno[2,3-*d*]pyrimidin-4-ylamino)-3-ethoxy-benzamide and
n) (6-Bromo-5-methyl-thieno[2,3-*d*]pyrimidin-4-yl)-(4-fluoro-2-isopropoxy-phenyl)-amine,
or a salt thereof.

10. A pharmaceutically acceptable salt of a compound according to any one of claims 1 to 9.

11. Pharmaceutical composition comprising a compound according to any one of claims 1 to 9 or a salt according to claim 10 and optionally a pharmaceutically acceptable carrier.

12. Pharmaceutical composition according to claim 11 further comprising an additional therapeutic agent.

13. Pharmaceutical composition according to claim 12 wherein the additional therapeutic agent is selected from an antidiabetic agent, a lipid lowering agent, a cardiovascular agent, an antihypertensive agent, a diuretic agent, a thrombocyte aggregation inhibitor, an antineoplastic agent or an anti-obesity agent.

14. Compound as defined in any one of claims 1 to 9 or a salt according to claim 10 for use as a medicament.

15. Compound as defined in any one of claims 1 to 9 or a salt according to claim 10 for use in the prophylaxis or therapy of metabolic diseases, hematopoietic disorders, neurodegenerative diseases, kidney damage, inflammatory disorders and cancer and their consecutive complications and diseases.

16. Compound according to any one of claims 1 to 9 or a salt according to claim 10 for use in the prophylaxis or therapy of metabolic diseases of the carbohydrate and/or lipid metabolism and their consecutive complications and disorders.

17. Compound according to any one of claims 1 to 9 or a salt according to claim 10 for use in the prophylaxis or therapy of diabetes.

18. Compound for use according to any one of claims 14 to 17, wherein the use comprises concomitant or sequential administration to a patient in combination with an additional therapeutic agent.

19. Compound as defined in any of claims 1 to 9 or a salt according to claim 10 for use in treating or preventing a cytokine related disorder selected from the group consisting of inflammatory diseases, autoimmune diseases, destructive bone disorders, proliferative disorders, infectious diseases, neurodegenerative diseases and allergies.

20. Compound for use according to claim 19, wherein the use comprises concomitant or sequential administration to a patient in combination with an additional therapeutic agent.

21. Compound for use according to claim 20, wherein the additional therapeutic agent is selected from a histamine antagonist, a bradikinin antagonist, serotonin antagonist, leukotriene, an anti-asthmatic, an NSAID, an antipyretic, a corticosteroid, an antibiotic, an analgetic, a uricosuric agent, chemotherapeutic agent, an anti gout agent, a bronchodilator, a cyclooxygenase-2 inhibitor, a steroid, a 5-lipoxygenase inhibitor, an immunosuppressive agent, a leukotriene antagonist, a cytostatic agent, an antineoplastic agent, a mTor inhibitor, a Tyrosine kinase inhibitor, antibodies or fragments thereof against cytokines and soluble parts (fragments) of cytokine receptors.

## Patentansprüche

1. Verbindung der allgemeinen Formel 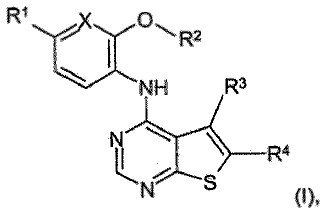 worin
X CH oder N darstellt,
R¹ ist ein Wasserstoff- oder Halogenatom, CN oder CONH₂,
R² ist eine geradkettige oder verzweigte C₁₋₆-Alkyl-Gruppe, die gegebenenfalls von Position zwei an mit ein oder zwei OH, C₁₋₄-Alkoxy oder NH₂ substituiert ist, oder die an irgendeiner Position mit ein bis drei F substituiert ist, oder mit einer CO₂H-, CONH₂-, CONH(C₁₋₃-Alkyl)-, CON(C₁₋₃-Alkyl)₂-, CONRR'-, SO₂NH₂-, SO₂NH(C₁₋₃-Alkyl)- oder SO₂N(C₁₋₃-Alkyl)₂-Gruppe,
wobei die Reste R und R', zusammen mit dem N-Atom, an das sie gebunden sind, einen 3-8-gliedrigen Ring bilden, worin eine CH₂-Gruppe durch O, S, SO, SO₂ oder N ersetzt sein kann, und worin irgendein Kohlenstoff-Atom, das nicht mit dem Stickstoff-Atom verknüpft ist, mit OH, F, C₁₋₃-Alkyl oder NH₂ substituiert sein kann;
eine C₃₋₈-Cycloalkyl-Gruppe, die gegebenenfalls mit ein oder zwei OH, C₁₋₄-Alkoxy oder NH₂ an irgendeinem Kohlenstoff-Atom substituiert ist, das nicht mit dem Sauerstoffatom verknüpft ist; oder mit F, CO₂H, CONH₂, CONH(C₁₋₃-Alkyl), CON(C₁₋₃-Alkyl)₂, SO₂NH₂, SO₂NH(C₁₋₃-Alkyl), SO₂N(C₁₋₃-Alkyl)₂, SO₂(C₁₋₃-Alkyl), (CH₂)ₘOR⁵; (CH₂)ₘN(R⁵)₂,
wobei die oben als Substituent für die Alkyl- und Cycloalkyl-Gruppen erwähnten NH₂-Gruppen gegebenenfalls unabhängig substituiert sein können mit C₁₋₃-Alkyl, -(CH₂)ₘ-OH, -(CH₂)ₘ-OCH₃, -(CH₂)ₘ-CN, -(CH₂)ₘ-F, SO₂(C₁₋₃-Alkyl), CO₂(C₁₋₄-Alkyl) oder CO(C₁₋₃-Alkyl);
oder ein Heterocyclylsystem, ausgewählt aus irgendeiner der folgenden Formeln: 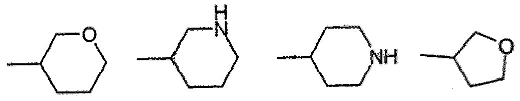 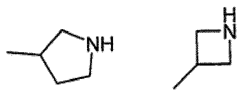 gegebenenfalls substituiert mit ein oder mehr R⁶,
worin das Wasserstoffatom der NH-Gruppe gegebenenfalls durch C₁₋₃-Alkyl, SO₂(C₁₋₃-Alkyl), CO₂(C₁₋₄-Alkyl) oder CO(C₁₋₃-Alkyl) ersetzt sein kann;
R³ ist eine C₁₋₂-Alkyl-Gruppe;
R⁴ ist F, Cl, Br, I oder CN;
R⁵ ist ausgewählt aus H und C₁₋₄-Alkyl;
R⁶ ist ausgewählt aus OH, OR⁵ und N(R⁵)₂ an irgendeinem Kohlenstoff-Atom, das nicht mit O oder N verknüpft ist; F; CO₂H; CON(R⁵)₂; SO₂N(R⁵)₂; SO₂R⁵; (CH₂)ₘOR⁵; (CH₂)ₘN(R⁵)₂; und
m ist 1, 2 oder 3;
oder ein Salz davon.

2. Verbindung der Formel 1 nach Anspruch 1, wobei
X CH oder N darstellt,
R¹ ist ein Wasserstoff- oder Fluoratom oder eine CONH₂-Gruppe,
R² ist eine geradkettige oder verzweigte C₁₋₄-Alkyl-Gruppe, die gegebenenfalls von Position 2 an mit NH₂ substituiert ist; oder eine geradkettige oder verzweigte C₁₋₄-Alkyl-Gruppe, die an irgendeiner Position mit ein bis drei F substituiert ist; oder eine geradkettige oder verzweigte C₁₋₄-Alkyl-Gruppe, die an irgendeiner Position mit CONH₂, CONH(C₁₋₃-Alkyl), CON(C₁₋₃-Alk_{Y}1)₂ oder CONRR' substituiert ist,
wobei die Reste R und R', zusammen mit dem N-Atom, an das sie gebunden sind, einen 3-8-gliedrigen Ring bilden, worin eine CH₂-Gruppe durch O oder N ersetzt sein kann, und worin irgendein Kohlenstoff-Atom mit OH, F oder NH₂ substituiert sein kann;
eine C₅₋₇-Cycloalkyl-Gruppe, die gegebenenfalls mit ein oder zwei OH, C₁₋₄-Alkoxy oder NH₂ an irgendeinem Kohlenstoff-Atom, das nicht an das Sauerstoffatom gebunden ist, substituiert ist,
wobei die oben als Substituent für die Alkyl- und Cycloalkyl-Gruppen erwähnten NH₂-Gruppen gegebenenfalls unabhängig mit C₁₋₃-Alkyl, -(CH₂)ₘ-OH, -(CH₂)ₘ-OCH₃, -(CH2)ₘ-CN, -(CH₂)ₘ-F, SO₂(C₁₋₃-Alkyl), CO₂(C₁₋₄-Alkyl) oder CO(C₁₋₃-Alkyl) substituiert sein können;
oder ein Heterocyclyl-System, ausgewählt aus irgendeiner der folgenden Formeln: 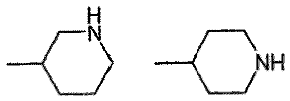 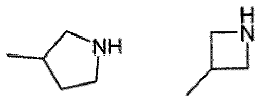 worin das Wasserstoffatom der NH-Gruppe gegebenenfalls durch SO₂(C₁₋₃-Alkyl) oder CO₂(C₁₋₄-Alkyl) ersetzt sein kann;
R³ ist eine C₁₋₂-Alkyl-Gruppe;
R⁴ ist F, Cl, Br oder CN; und
m ist 1, 2 oder 3;
oder ein Salz davon.

3. Verbindung der Formel I nach Anspruch 2, worin
X CH oder N darstellt,
R¹ ist ein Wasserstoff- oder Fluoratom oder eine CONH₂-Gruppe,
R² ist eine geradkettige oder verzweigte C₁₋₄-Alkyl-Gruppe, die gegebenenfalls von Position 2 an mit NH₂ substituiert ist; oder die an irgendeiner Position mit ein bis drei F substituiert ist;
eine Cyclohexyl-Gruppe, die gegebenenfalls mit einem OH, C₁₋₃-Alkoxy oder NH₂ an irgendeinem Kohlenstoff-Atom, das nicht an das Sauerstoffatom gebunden ist, substituiert ist,
wobei die oben als Substituent für die Alkyl- und Cycloalkyl-Gruppen erwähnten NH₂-Gruppen gegebenenfalls unabhängig mit C₁₋₃-Alkyl, SO₂(C₁₋₃-Alkyl), CO₂(C₁₋₄-Alkyl) oder CO(C₁₋₃-Alkyl) substituiert sein können;
oder ein Heterocyclyl-System, ausgewählt aus irgendeiner der folgenden Formeln: 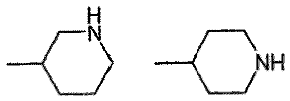 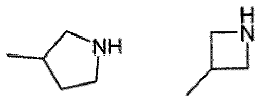 worin das Wasserstoffatom der NH-Gruppe gegebenenfalls durch SO₂(C₁₋₃-Alkyl) oder CO₂(C₁₋₄-Alkyl) ersetzt sein kann;
R³ ist eine C₁₋₂-Alkyl-Gruppe und
R⁴ ist F, Cl oder Br,
oder ein Salz davon.

4. Verbindung der Formel I nach irgendeinem der Ansprüche 1 bis 3, wobei X und R¹, R² und R⁴ wie in den vorhergehenden Ansprüchen definiert sind und R³ ist CH₃,
oder ein Salz davon.

5. Verbindung der Formel I nach irgendeinem der Ansprüche 1 bis 3, wobei R² bis R⁴ wie in den vorhergehenden Ansprüchen definiert sind,
X ist CH und
R¹ ist F oder CONH₂,
oder ein Salz davon.

6. Verbindung der Formel I nach irgendeinem der Ansprüche 1 bis 3, wobei R² bis R⁴ wie in den vorhergehenden Ansprüchen definiert sind,
X ist N und
R¹ ist H,
oder ein Salz davon.

7. Verbindung der Formel I nach irgendeinem der Ansprüche 1 bis 3, wobei X und R¹ bis R³ wie in den vorhergehenden Ansprüchen definiert sind und
R⁴ ist F, Cl oder Br,
oder ein Salz davon.

8. Verbindung der Formel I nach Anspruch 7, wobei
X und R¹ bis R³ wie in den vorhergehenden Ansprüchen definiert sind und
R⁴ ist Cl oder Br,
oder ein Salz davon.

9. Verbindung, ausgewählt aus:
a) (trans-3-[2-(6-Chlor-5-methyl-thieno[2,3-*d*]pyrimidin-4-ylamino)-5-fluor-phenoxy]-cyclohexanol,
b) (6-Chlor-5-methyl-thieno[2,3-*d*]pyrimidin-4-yl)-[2-(trans-4-methylamino-cyclohexyl-oxy)-pyridin-3-yl]-amin,
c) (6-Chlor-5-methyl-thieno[2,3-*d*]pyrimidin-4-yl)-[4-fluor-2-(2-fluor-1-fluormethyl-ethoxy)-phenyl]-amin,
d) (6-Chlor-5-methyl-thieno[2,3-*d*]pyrimidin-4-yl)-[4-fluor-2-((R)-piperidin-3-yloxy)-phenyl]-amin,
e) 6-Chlor-5-methyl-thieno[2,3-*d*]pyrimidin-4-yl)-[4-fluor-2-((R)-1-methansulfonyl-pi-peridin-3-yloxy)-phenyl]-amin,
f) [2-(2-Amino-1-methyl-ethoxy)-4-fluor-phenyl]-(6-brom-5-methyl-thieno[2,3-*d*]pyri-midin-4-yl)-amin,
g) *N*-{2-[2-(6-Brom-5-methyl-thieno[2,3-*d*]pyrimidin-4-ylamino)-5-fluor-phenoxy]-propyl}-methansulfonamid,
h) (6-Brom-5-methyl-hieno[2,3-*d*]pyrimidin-4-yl)-[4-fluor-2-(tetrahydro-pyran-4-yl-oxy)-phenyl]-amin,
i) [2-(2-Amino-1-methyl-ethoxy)-4-fluor-phenyl]-(6-chlor-5-methyl-thieno[2,3-*d*]pyri-midin-4-yl)-amin,
j) *N*-{2-[2-(6-Chlor-5-methyl-thieno[2,3-*d*]pyrimidin-4-ylamino)-5-fluor-phenoxy]-pro-pyl}-acetamid,
k) *N*-{2-[2-(6-Chlor-5-methyl-thieno[2,3-*d*]pyrimidin-4-ylamino)-5-fluor-phenoxy]-pro-pyl}-methansulfonamid,
l) 4-(6-Chlor-5-methyl-thieno[2,3-*d*]pyrimidin-4-ylamino)-3-ethoxy-benzamid,
m) 4-(6-Brom-5-methyl-thieno[2,3-*d*]pyrimidin-4-ylamino)-3-ethoxy-benzamid und
n) (6-Brom-5-methyl-thieno[2,3-*d*]pyrimidin-4-yl)-(4-fluor-2-isopropoxy-phenyl)-amin oder ein Salz davon.

10. Pharmazeutisch akzeptables bzw. annehmbares Salz einer Verbindung nach irgendeinem der Ansprüche 1 bis 9.

11. Pharmazeutische Zusammensetzung, umfassend eine Verbindung nach irgendeinem der Ansprüche 1 bis 9 oder ein Salz nach Anspruch 10 und gegebenenfalls einen pharmazeutisch akzeptablen bzw. annehmbaren Träger.

12. Pharmazeutische Zusammensetzung nach Anspruch 11, weiterhin umfassend ein zusätzliches therapeutisches Mittel.

13. Pharmazeutische Zusammensetzung nach Anspruch 12, wobei das zusätzliche therapeutische Mittel ausgewählt ist aus einem Antidiabetesmittel, einem Lipid-senkenden Mittel, einem kardiovaskulären Mittel, einem Antibluthochdruckmittel, einem Diuretikum, einem Thrombozytenaggregationshemmer, einem antineoplastischen Mittel oder einem Mittel gegen Adipositas.

14. Verbindung wie definiert in irgendeinem der Ansprüche 1 bis 9 oder ein Salz nach Anspruch 10 zur Verwendung als Medikament.

15. Verbindung wie definiert in irgendeinem der Ansprüche 1 bis 9 oder ein Salz nach Anspruch 10 zur Verwendung in der Prophylaxe oder Therapie von metabolischen Erkrankungen, hämatopoietischen Störungen, neurodegenerativen Erkrankungen, Nierenschäden, inflammatorschen Störungen und Krebs und ihren Folgekomplikationen und -erkrankungen.

16. Verbindung nach irgendeinem der Ansprüche 1 bis 9 oder ein Salz nach Anspruch 10 zur Verwendung in der Prophylaxe oder Therapie von metabolischen Erkrankungen des Kohlenhydrat- und/oder Lipid-Metabolismus und ihren Folgekomplikationen und -Störungen.

17. Verbindung nach irgendeinem der Ansprüche 1 bis 9 oder ein Salz nach Anspruch 10 zur Verwendung in der Prophylaxe oder Therapie von Diabetes.

18. Verbindung zur Verwendung nach irgendeinem der Ansprüche 14 bis 17, wobei die Verwendung die gleichzeitige oder aufeinander folgende Verabreichung an einen Patienten in Kombination mit einem zusätzlichen therapeutischen Mittel umfasst.

19. Verbindung wie definiert in irgendeinem der Ansprüche 1 bis 9 oder ein Salz nach Anspruch 10 zur Verwendung in der Behandlung oder Vorbeugung einer mit Cytokin in Zusammenhang stehende Störung, ausgewählt aus der Gruppe, bestehend aus inflammatorischen Erkrankungen, Autoimmunerkrankungen, destruktiven Knochenstörungen, proliferativen Störungen, Infektionserkrankungen, neurodegenerativen Erkrankungen und Allergien.

20. Verbindung zur Verwendung nach Anspruch 19, wobei die Verwendung die gleichzeitige oder aufeinander folgende Verabreichung an einen Patienten in Kombination mit einem zusätzlichen therapeutischen Mittel umfasst.

21. Verbindung zur Verwendung nach Anspruch 20, wobei das zusätzliche therapeutische Mittel ausgewählt ist aus einem Histaminantagonisten, einem Bradikininantagonisten, Serotoninantagonist, Leukotrien, einem Anti-Asthmatikum, einem NSAID, einem Antipyretikum, einem Kortikosteroid, einem Antibiotikum, einem Analgetikum, einem Urikosurikum, einem chemotherapeutischen Mittel, einem Anti-Gicht-Mittel, einem Bronchodilator, einem Cyclooxygenase-2-Inhibitor, einem Steroid, einem 5-Lipoxygenase-Inhibitor, einem immunsuppressiven Mittel, einem Leukotrienantagonisten, einem zytostatischen Mittel, einem antineoplastischen Mittel, einem mTor-Inhibitor, einem Tyrosinkinase-Inhibitor, Antikörpern oder Fragmenten hiervon gegen Cytokine und lösliche Teile (Fragmente) von Cytokinrezeptoren.

## Revendications

1. Composé de formule générale 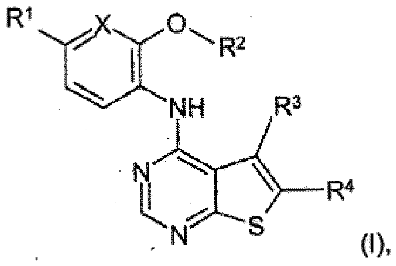 dans laquelle
X est CH ou N,
R¹ est un hydrogène ou un atome d'halogène, CN ou CONH₂,
R² est un groupe alkyle en C₁ à C₆ à chaîne linéaire ou ramifiée qui est éventuellement substitué à partir de la position 2 et sur les suivantes avec un ou deux OH, alcoxy en C₁ à C₄ ou NH₂, ou qui est substitué sur n'importe quelle position avec un à trois F, ou avec un groupe CO₂H, CONH₂, CONH(alkyle en C₁ à C₃), CON(alkyle en C₁ à C₃)₂, CONRR', SO₂NH₂, SO₂NH(alkyle en C₁ à C₃) ou SO₂N(alkyle en C₁ à C₃)₂,
dans lequel les restes R et R' ensemble avec l'atome N auquel ils sont liés forment un cycle de 3 à 8 chaînons, dans lequel un groupe CH₂ peut être remplacé par 0, S, SO, SO₂ ou N, et dans lequel n'importe quel atome de carbone différent de celui fixé à l'atome d'azote peut être substitué avec OH, F, un alkyle en C₁ à C₃ ou NH₂ ;
un groupe cycloalkyle en C₃ à C₈ qui est éventuel-lement substitué avec un ou deux OH, alcoxy en C₁ à C₄ ou NH₂ sur n'importe quel atome de carbone autre que celui fixé à l'atome d'oxygène ; ou avec F, CO₂H, CONH₂, un CONH(alkyle en C₁ à C₃), un CON(alkyle en C₁ à C₃)₂, SO₂NH₂, un SO₂NH(alkyle en C₁ à C₃), un SO₂N(alkyle en C₁ à C₃)₂, un SO₂(alkyle en C₁ à C₃), (CH₂)ₘOR⁵ ; (CH₂)ₘN(R⁵)₂,
dans lequel les groupes NH₂ mentionnés ci-dessus en tant que substituant pour les groupes alkyle et cycloalkyle peuvent éventuellement être indépendamment substitués avec un alkyle en C₁ à C₃, -(CH₂)ₘ-OH, -(CH₂)ₘ-OCH₃, -(CH₂)ₘ-CN, -(CH₂)ₘ-F, un SO₂-(alkyle en C₁ à C₃), un CO₂ (alkyle en C₁ à C₄) ou un CO(alkyle en C₁ à C₃) ;
ou un système hétérocyclyle sélectionné parmi l'une quelconque des formules suivantes : 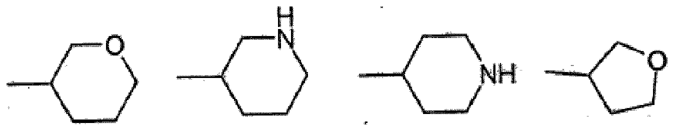 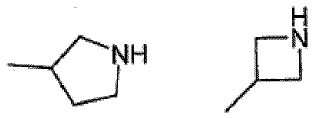 éventuellement substitué avec un ou plusieurs R⁶,
dans lequel l'atome d'hydrogène du groupe NH peut être remplacé éventuellement par un alkyle en C₁ à C₃, un SO₂(alkyle en C₁ à C₃), un CO₂ (alkyle en C₁ à C₄) ou un CO(alkyle en C₁ à C₃) ;
R³ est un groupe alkyle en C₁ à C₂ ;
R⁴ est F, Cl, Br, I ou CN ;
R⁵ est sélectionné parmi H et un alkyle en C₁ à C₄ ;
R⁶ est sélectionné parmi OH, OR⁵ et N(R⁵)₂ sur n'importe quel atome de carbone différent de celui fixé à 0 ou à N ; F ; CO₂H ; CON(R⁵) ₂ ; SO₂N(R⁵)₂ ; SO0₂R⁵ ; (CH₂)ₘOR⁵ _{;} (CH₂)ₘN(R⁵)₂ ; et
m est 1, 2 ou 3 ;
ou un sel de celui-ci.

2. Composé de formule I selon la revendication 1, dans lequel
X est CH ou N,
R¹ est un hydrogène ou un atome de fluor ou un groupe CONH₂,
R² est un groupe alkyle en C₁ à C₄ à chaîne linéaire ou ramifiée qui est éventuellement substitué à partir de la position 2 et sur les suivantes avec NH₂ ; ou un groupe alkyle en C₁ à C₄ à chaîne linéaire ou ramifiée qui est substitué sur n'importe quelle position avec un à trois F ; ou un groupe alkyle en C₁ à C₄ à chaîne linéaire ou ramifiée qui est substitué sur n'importe quelle position avec CONH₂, CONH(alkyle en C₁ à C₃), CON(alkyle en C₁ à C₃)₂ ou CONRR',
dans lequel les restes R et R' ensemble avec l'atome N auquel ils sont liés forment un cycle de 3 à 8 chaînons, dans lequel un groupe CH₂ peut être remplacé par 0 ou N, et dans lequel n'importe quel atome de carbone peut être substitué avec OH, F ou NH₂ ;
un groupe cycloalkyle en C₅ à C₇ qui est éventuellement substitué avec un ou deux OH, alcoxy en C₁ à C₄ ou NH₂ sur n'importe quel atome de carbone autre que celui fixé à l'atome d'oxygène ;
dans lequel les groupes NH₂ mentionnés ci-dessus en tant que substituant pour les groupes alkyle et cycloalkyle peuvent éventuellement être indépendamment substitués avec un alkyle en C₁ à C₃, -(CH₂)ₘ-OH, -(CH₂)ₘ-OCH₃, -(CH₂)ₘ-CN, -(CH₂)ₘ-F, un SO₂-(alkyle en C₁ à C₃), un CO₂(alkyle en C₁ à C₄) ou un CO(alkyle en C₁ à C₃) ;
ou des systèmes hétérocyclyles sélectionnés parmi l'une quelconque des formules suivantes : 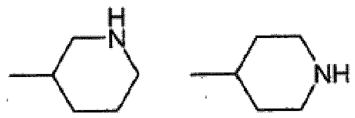 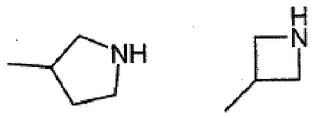 dans lesquels l'atome d'hydrogène du groupe NH peut éventuellement être remplacé par un SO₂(alkyle en C₁ à C₃) ou un CO₂(alkyle en C₁ à C₄) ;
R³ est un groupe alkyle en C₁ à C₂ ;
R⁴ est F, Cl, Br ou CN ; et
m est 1, 2 ou 3 ;
ou un sel de celui-ci.

3. Composé de formule I selon la revendication 2, dans lequel
X est CH ou N,
R¹ est un hydrogène ou un atome de fluor ou un groupe CONH₂,
R² est un groupe alkyle en C₁ à C₄ à chaîne linéaire ou ramifiée qui est éventuellement substitué à partir de la position 2 et sur les suivantes avec NH₂, ou qui est substitué sur n'importe quelle position avec un à trois F ;
un groupe cyclohexyle qui est éventuellement substitué avec OH, un alcoxy en C₁ à C₃ ou NH₂ sur n'importe quel atome de carbone différent de celui fixé à l'atome d'oxygène,
dans lequel les groupes NH₂ mentionnés ci-dessus en tant que substituant pour les groupes alkyle et cycloalkyle peuvent éventuellement être indépendamment substitués avec un alkyle en C₁ à C₃, un SO₂(alkyle en C₁ à C₃), un CO₂(alkyle en C₁ à C₄) ou un CO-(alkyle en C₁ à C₃) ;
ou des systèmes hétérocyclyles sélectionnés parmi l'une quelconque des formules suivantes : 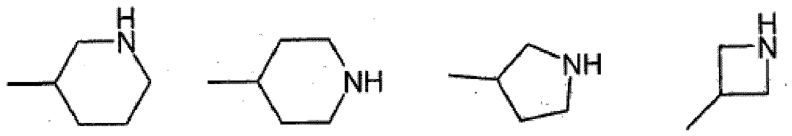 dans lesquels l'atome d'hydrogène du groupe NH peut éventuellement être remplacé par un SO₂(alkyle en C₁ à C₃) ou un CO₂(alkyle en C₁ à C₄) ;
R³ est un groupe alkyle en C₁ à C₂, et
R⁴ est F, Cl ou Br,
ou un sel de celui-ci.

4. Composé de formule I selon l'une quelconque des revendications 1 à 3, dans lequel
X et R¹, R² et R⁴ sont tels que définis dans les revendications précédentes et
R³ est CH₃,
ou un sel de celui-ci.

5. Composé de formule I selon l'une quelconque des revendications 1 à 3, dans lequel
R² à R⁴ sont tels que définis dans les revendications précédentes,
X est CH et
R¹ est F ou CONH₂,
ou un sel de celui-ci.

6. Composé de formule I selon l'une quelconque des revendications 1 à 3, dans lequel
R² à R⁴ sont tels que définis dans les revendications précédentes,
X est N et
R¹ est H,
ou un sel de celui-ci.

7. Composé de formule I selon l'une quelconque des revendications 1 à 3, dans lequel
X et R¹ à R³ sont tels que définis dans les revendications précédentes, et
R⁴ est F, Cl ou Br,
ou un sel de celui-ci.

8. Composé de formule I selon la revendication 7, dans lequel
X et R¹ à R³ sont tels que définis dans les revendications précédentes, et
R⁴ est Cl ou Br,
ou un sel de celui-ci.

9. Composé sélectionné parmi :
a) le (trans-3-[2-(6-chloro-5-méthylthiéno[2,3-d] pyrimidin-4-ylamino)-5-fluorophénoxy]-cyclohexanol,
b) la (6-chloro-5-méthylthiéno[2,3-d]pyrimidin-4-yl)-[2-(trans-4-méthylaminocyclohexyloxy)-pyridin-3-yl]-amine,
c) la (6-chloro-5-méthylthiéno[2,3-d]pyrimidin-4-yl)-[4-fluoro-2-(2-fluoro-1-fluorométhyléthoxy)-phényl]-amine,
d) la (6-chloro-5-méthylthiéno[2,3-d]pyrimidin-4-yl)-[4-fluoro-2-((R)-pipéridin-3-yloxy)-phényl]-amine,
e) la 6-chloro-5-méthylthiéno[2,3-d]pyrimidin-4-yl)-[4-fluoro-2-((R)-1-méthanesulfonylpipéridin-3-yloxy)-phényl]-amine,
f) la [2-(2-amino-1-méthyléthoxy)-4-fluorophényl]-(6-bromo-5-méthylthiéno[2,3-d]pyrimidin-4-yl)-amine,
g) le N-{2-[2-(6-bromo-5-méthylthiéno[2,3-dpyrimidin-4-ylamino)-5-fluorophénoxy]-propyl}-méthane-sulfonamide,
h) la (6-bromo-5-méthylthiéno[2,3-d]pyrimidin-4-yl)-[4-fluoro-2-(tétrahydropyran-4-yloxy)-phényl]-amine,
i) la [2-(2-amino-1-méthyléthoxy)-4-fluorophényl]-(6-chloro-5-méthylthiéno[2,3-d]pyrimidin-4-yl)-amine,
j) le N-{2-[2-(6-chloro-5-méthylthiéno[2,3-d]-pyrimidin-4-ylamino)-5-fluorophénoxy]-propyl}-acétamide,
k) le N-{2-[2-(6-chloro-5-méthylthiéno[2,3-d] pyrimidin-4-ylamino)-5-fluorophénoxy]-propyl}-méthane-sulfonamide,
l) le 4-(6-chloro-5-méthylthiéno[2,3-d]pyrimidin-4-ylamino)-3-éthoxybenzamide,
m) le 4-(6-bromo-5-méthylthiéno[2,3-d]pyrimidin-4-ylamino)-3-éthoxybenzamide, et
n) la (6-bromo-5-méthylthiéno[2,3-d]pyrimidin-4-yl)-(4-fluoro-2-isopropoxyphényl)-amine,
ou un sel de ceux-ci.

10. Sel pharmaceutiquement acceptable d'un composé selon l'une quelconque des revendications 1 à 9.

11. Composition pharmaceutique, comprenant un composé selon l'une quelconque des revendications 1 à 9 ou un sel selon la revendication 10 et éventuellement un support pharmaceutiquement acceptable.

12. Composition pharmaceutique selon la revendication 11, comprenant en outre un agent thérapeutique supplémentaire.

13. Composition pharmaceutique selon la revendication 12, dans laquelle l'agent thérapeutique supplémentaire est sélectionné parmi un agent antidiabétique, un agent hypolipémiant, un agent cardio-vasculaire, un agent antihypertenseur, un agent diurétique, un inhibiteur de l'agrégation des thrombocytes, un agent antinéoplasique et un agent anti-obésité.

14. Composé tel que défini dans l'une quelconque des revendications 1 à 9 ou sel selon la revendication 10, pour une utilisation comme médicament.

15. Composé tel que défini dans l'une quelconque des revendications 1 à 9 ou sel selon la revendication 10, pour une utilisation dans la prophylaxie ou le traitement des maladies métaboliques, des troubles hématopoïétiques, des maladies neurodégénératives, des lésions rénales, des troubles inflammatoires et du cancer et de leurs complications et maladies consécutives.

16. Composé tel que défini dans l'une quelconque des revendications 1 à 9 ou sel selon la revendication 10, pour une utilisation dans la prophylaxie ou le traitement des maladies métaboliques du métabolisme des glucides et/ou des lipides et de leurs complications et troubles consécutifs.

17. Composé tel que défini dans l'une quelconque des revendications 1 à 9 ou sel selon la revendication 10, pour une utilisation dans la prophylaxie ou le traitement du diabète.

18. Composé pour une utilisation selon l'une quelconque des revendications 14 à 17, dans lequel l'utilisation comprend une administration concomitante ou séquentielle à un patient en combinaison avec un agent thérapeutique supplémentaire.

19. Composé tel que défini dans l'une quelconque des revendications 1 à 9 ou sel selon la revendication 10, pour une utilisation dans le traitement ou la prévention d'un trouble associé aux cytokines sélectionné dans le groupe consistant en les maladies inflammatoires, les maladies auto-immunes, les troubles destructifs de l'os, les maladies prolifératives, les maladies infectieuses, les maladies neurodégénératives et les allergies.

20. Composé pour une utilisation selon la revendication 19, dans lequel l'utilisation comprend une administration concomitante ou séquentielle à un patient en combinaison avec un agent thérapeutique supplémentaire.

21. Composé pour une utilisation selon la revendication 20, dans lequel l'agent thérapeutique supplémentaire est sélectionné parmi un antagoniste de l'histamine, un antagoniste de la bradikinine, un antagoniste de la sérotonine, un leucotriène, un antiasthmatique, un AINS, un antipyrétique, un corticostéroïde, un antibiotique, un analgésique, un agent uricosurique, un agent chimiothérapeutique, un agent anti-goutte, un bronchodilatateur, un inhibiteur de cyclooxygénase-2, un stéroïde, un inhibiteur de 5-lipoxygénase, un agent immunosuppresseur, un antagoniste de leucotriène, un agent cytostatique, un agent antinéoplasique, un inhibiteur de mTor, un inhibiteur de tyrosine kinase, des anticorps ou des fragments de ceux-ci dirigés contre les cytokines et les parties solubles (fragments) des récepteurs des cytokines.
